(19) Europäisches Patentamt
European Patent Office
Office européen des brevets

(11) Veröffentlichungsnummer : **0 473 551 A1**

(12) # EUROPÄISCHE PATENTANMELDUNG

(21) Anmeldenummer : **91810671.7**

(22) Anmeldetag : **22.08.91**

(51) Int. Cl.[5] : **C07D 239/54,** C07D 239/52, C07D 239/96, C07D 239/70, A01N 43/54

(30) Priorität : **31.08.90 CH 2832/90**

(43) Veröffentlichungstag der Anmeldung : **04.03.92 Patentblatt 92/10**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Suchy, Milos, Dr.**
**Violaweg 71**
**CH-4303 Kaiseraugst (CH)**
Erfinder : **Wenger, Jean, Dr.**
**Brunnenwiesenstrasse 1**
**CH-8610 Uster (CH)**
Erfinder : **Winternitz, Paul, Dr.**
**Am Pfisterhölzli 50**
**CH-8606 Greifensee (CH)**
Erfinder : **Zeller, Martin, Dr.**
**Kronengasse 7**
**CH-5400 Baden (CH)**

(54) **3-Aryluracil-Derivate, Verfahren zu ihrer Herstellung und diese enthaltende Unkrautbekämpfungsmittel.**

(57) Die Erfindung betrifft 3-Aryluracile der allgemeinen Formel

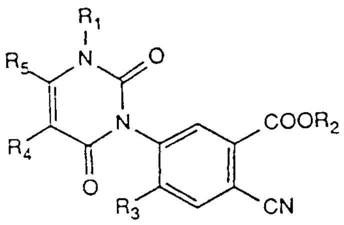

worin
$R_1$ Wasserstoff, $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl,
$R_2$ Wasserstoff, $C_1$-$C_6$-Alkyl, $C_2$-$C_6$-Alkoxyalkyl, gegebenenfalls mit 1 bis 3 $C_1$-$C_3$Alkylgruppen ringsubstituiertes $C_4$-$C_7$-Cycloalkenyl-$C_1$-$C_4$-alkyl, $C_3$-$C_4$-Alkenyl, gegebenenfalls mit 1 bis 3 $C_1$-$C_3$-Alkylgruppen ringsubstituiertes $C_4$-$C_7$-Cycloalkenyl-$C_3$-$C_5$-alkenyl, Aryl-$C_3$-$C_5$-alkenyl, $C_3$-$C_4$-Alkinyl, gegebenenfalls mit 1 bis 3 $C_1$-$C_3$-Alkylgruppen ringsubstituiertes $C_4$-$C_7$-Cycloalkenyl-$C_3$-$C_5$-alkinyl oder Aryl-$C_3$-$C_5$-alkinyl,
$R_3$ Wasserstoff oder Halogen,
$R_4$ Wasserstoff, Halogen oder $C_1$-$C_4$-Alkyl und
$R_5$ $C_1$-$C_4$-Alkyl oder $C_1$-$C_4$-Halogenalkyl, oder
$R_4$ und $R_5$ zusammen Tri- oder Tetramethylen bedeuten, mit der Massgabe, dass, falls $R_5$ für $C_1$-$C_4$-Halogenalkyl steht, $R_1$ verschieden von $C_1$-$C_4$-Halogenalkyl und $R_2$ verschieden von Wasserstoff ist, und die entsprechenden Enoläther derjenigen Verbindungen der Formel 1, in denen $R_1$ von Wasserstoff oder $C_1$-$C_4$-Halogenalkyl verschieden ist, sowie Salze derjenigen Verbindungen der Formel I, in denen $R_1$ und/oder $R_2$ Wasserstoff bedeutet.
Die erfindungsgemässen Verbindungen, d.h. die Verbindungen der Formel I und deren Enoläther und Salze, sind herbizid wirksam und eignen sich als Wirkstoffe von Unkrautbekämpfungsmitteln. Somit umfasst die Erfindung auch Unkrautbekämpfungsmittel, welche erfindungsgemässe Verbindungen als Wirkstoffe enthalten, Verfahren zur Herstellung dieser Verbindungen sowie die Verwendung der Verbindungen bzw. Mittel zur Bekämpfung von Unkräutern.

EP 0 473 551 A1

Die vorliegende Erfindung betrifft heterocyclische Verbindungen, und zwar 3-Aryluracile der allgemeinen Formel

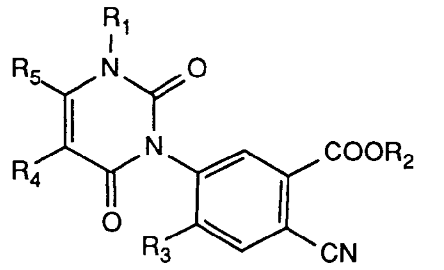

I

worin
$R_1$ Wasserstoff, $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl,
$R_2$ Wasserstoff, $C_1$-$C_6$-Alkyl, $C_2$-$C_6$-Alkoxyalkyl, gegebenenfalls mit 1 bis 3 $C_1$-$C_3$-Alkylgruppen ringsubstituiertes $C_4$-$C_7$-Cycloalkenyl-$C_1$-$C_4$-alkyl, $C_3$-$C_4$-Alkenyl, gegebenenfalls mit 1 bis 3 $C_1$-$C_3$-Alkylgruppen ringsubstituiertes $C_4$-$C_7$-Cycloalkenyl-$C_3$-$C_5$-alkenyl, Aryl-$C_3$-$C_5$-alkenyl, $C_3$-$C_4$-Alkinyl, gegebenenfalls mit 1 bis 3 $C_1$-$C_3$-Alkylgruppen ringsubstituiertes $C_4$-$C_7$-Cycloalkenyl-$C_3$-$C_5$-alkinyl, oder Aryl-$C_3$-$C_5$-alkinyl,
$R_3$ Wasserstoff oder Halogen,
$R_4$ Wasserstoff, Halogen oder $C_1$-$C_4$-Alkyl und
$R_5$ $C_1$-$C_4$-Alkyl oder $C_1$-$C_4$-Halogenalkyl, oder
$R_4$ und $R_5$ zusammen Tri- oder Tetramethylen bedeuten, mit der Massgabe, dass, falls $R_5$ für $C_1$-$C_4$-Halogenalkyl steht, $R_1$ verschieden von $C_1$-$C_4$-Halogenalkyl und $R_2$ verschieden von Wasserstoff ist,
und die entsprechenden Enoläther derjenigen Verbindungen der Formel 1, in denen $R_1$ von Wasserstoff oder $C_1$-$C_4$-Halogenalkyl verschieden ist, sowie Salze derjenigen Verbindungen der Formel I, in denen $R_1$ und/oder $R_2$ Wasserstoff bedeutet.

Unter den oben erwähnten Enoläthern sind also die Verbindungen der Formel

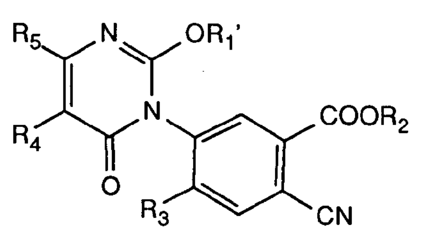

Ia

worin $R_1$'$C_1$-$C_4$-Alkyl, $C_3$-$C_4$-Alkenyl, $C_3$-$C_4$-Alkinyl oder $C_2$-$C_6$-Alkoxyalkyl bedeutet, zu verstehen.

Die erfindungsgemässen Verbindungen, d.h. die Verbindungen der Formel I und deren Enoläther und Salze, sind herbizid wirksam und eignen sich als Wirkstoffe von Unkrautbekämpfungsmitteln. Somit umfasst die Erfindung auch Unkrautbekämpfungsmittel, welche erfindungsgemässe Verbindungen als Wirkstoffe enthalten, Verfahren zur Herstellung dieser Verbindungen sowie die Verwendung der Verbindungen bzw. Mittel zur Bekämpfung von Unkräutern.

In der obigen Formel I umfasst "Halogen" Fluor, Chlor, Brom und Jod. Die Alkyl-, Alkenyl- und Alkinylreste können geradkettig oder verzweigt sein, wobei dies auch für den bzw. jeden Alkyl-, Alkenyl- bzw. Alkinylteil der verschiedenen Alkyl-, Alkenyl- bzw. Alkinyl-enthaltenden Gruppen, wie beispielsweise "gegebenenfalls mit 1 bis 3 $C_1$-$C_3$-Alkylgruppen ringsubstituiertes $C_4$-$C_7$-Cycloalkenyl-$C_3$-$C_5$-alkenyl", gilt. Eine Halogenalkylgruppe kann eine oder mehrere gleiche oder verschiedene Halogenatome aufweisen. Unter Aryl (der Aryl-$C_3$-$C_5$-alkenyl- und Aryl-$C_3$-$C_5$-alkinylgruppen) ist insbesondere Phenyl zu verstehen, wobei solche Gruppen substituiert sein können, und zwar mit 1 bis 3 Halogen, $C_1$-$C_3$-Alkyl, $C_1$-$C_3$-Halogenalkyl, $C_1$-$C_3$-Alkoxy, $C_1$-$C_3$-Alkylthio, $C_2$-$C_4$-Alkanoyl, $C_2$-$C_4$-Alkoxycarbonyl, $C_1$-$C_3$-Alkylsulfonyl, Nitro und/oder Cyano. Die von $R_4$ und $R_5$ gebildeten ankondensierten Ringe ($R_4$ und $R_5$ bedeuten zusammen Tri- oder Tetramethylen) sind durch die nachfolgenden Teilstrukturen dargestellt:

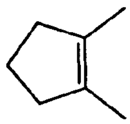 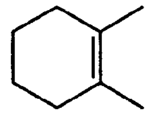

Bei den Salzen der Verbindungen der Formel I handelt es sich insbesondere um Alkalimetallsalze, z.B. Natrium- und Kaliumsalze; Erdalkalimetallsalze, z.B. Calcium- und Magnesiumsalze; Ammoniumsalze, d.h. unsubstituierte Ammoniumsalze und mono- oder mehrfach-substituierte Ammoniumsalze, z.B. Triäthylammonium- und Methylammoniumsalze, sowie um Salze mit anderen organischen Basen, z.B. mit Pyridin.

Das mögliche Vorhandensein mindestens eines asymmetrischen Kohlenstoffatoms in den Verbindungen der Formel I hat zur Folge, dass die Verbindungen in optisch isomeren Formen auftreten können. Durch das Vorliegen einer allfälligen aliphatischen C=C-Doppelbindung kann auch geometrische Isomerie auftreten. Die Formel I soll all diese möglichen isomeren Formen sowie Gemische davon umfassen.

Bedeutet $R_1$ oder $R_2$ Alkenyl oder Alkinyl, ist diese Gruppe vorzugsweise Allyl oder 3-Buten-2-yl bzw. Propargyl oder 3-Butin-2-yl. Im allgemeinen ist ein allfällig vorkommendes Halogenatom vorzugsweise Fluor.

Unabhängig voneinander bedeuten $R_1$ vorzugsweise $C_1$-$C_4$-Alkyl, insbesondere Methyl; $R_2$ vorzugsweise $C_1$-$C_6$-Alkyl, $C_2$-$C_6$-Alkoxyalkyl, $C_3$-$C_4$-Alkenyl oder $C_3$-$C_4$-Alkinyl; $R_3$ vorzugsweise Wasserstoff oder Fluor; $R_4$ vorzugsweise Wasserstoff, Methyl oder Fluor; und $R_5$ vorzugsweise $C_1$-$C_4$-Alkyl oder Trifluormethyl. Ebenfalls bevorzugt bedeuten $R_4$ und $R_5$ zusammen Tri- oder Tetramethylen.

Besonders bevorzugte Verbindungen der Formel I sind:
2-Cyano-5-(1,2,4,5,6,7-hexahydro-1-methyl-2,4-dioxo-3H-cyclopenta[d]pymidin-3-yl)-benzoesäure-isopropylester,
2-Cyano-4-fluor-5-(1,2,4,5,6,7-hexahydro-1-methyl-2,4-dioxo-3H-cyclopenta[d]-pyrimidin-3-yl)-benzoesäure-isopropylester,
2-Cyano-5-[1,4,5,6,7,8-hexahydro-1-methyl-2,4-dioxo-3(2H)-chinazolinyl]-benzoesäure-isopropylester,
2-Cyano-4-fluor-5-[1,4,5,6,7,8-hexahydro-1-methyl-2,4-dioxo-3(2H)-chinazolinyl]-benzoesäure-isopropylester,
2-Cyano-5-[3,6-dihydro-3,4-dimethyl-2,6-dioxo-1(2H)-pyrimidinyl]-4-fluorbenzoesäure-isopropylester,
2-Cyano-5-[3,6-dihydro-3-methyl-4-trifluormethyl-2,6-dioxo-(1(2H)-pyrimidinyl]-benzoesäure-isopropylester und
2-Cyano-5-[3,6-dihydro-3-methyl-4-trifluormethyl-2,6-dioxo-1(2H)-pyrimidinyl]-4-fluor-benzoesäure-isopropylester,
sowie die entsprechenden Methyl-, Aethyl-, n-Propyl-, n-Butyl-, sek.Butyl-, Isobutyl-, tert.Butyl-, Methoxymethyl-, 1-Methoxyäthyl-, 2-Methoxyäthyl-, 1-Methoxypropyl-, 1 -Aethoxyäthyl-, 2-Methoxy-1-methyläthyl-, Allyl-, 1 -Methyl-2-propenyl-, 1-Aethyl-2-propenyl, 2-Butenyl-, Propargyl-, 2-Butinyl-, 1-Methyl-2-propinyl- und 1 -Aethyl-2-propinylester obiger Verbindungen.

Weitere Vertreter von Verbindungen der Formel I sind:
2-Cyano-4-fluor-5-[5-chlor-3,6-dihydro-4-methyl-2,6-dioxo-1(2H)-pyrimidinyl]-benzoesäure-isopropylester,
2-Cyano-4-fluor-5-[5-chlor-3,6-dihydro-3,4-dimethyl-2,6-dioxo-1(2H)-pyrimidinyl]-benzoesäure-isopropylester,
2-Cyano-4-fluor-5- [3,6-dihydro-4-trifluormethyl-2,6-dioxo-1(2H)-pyrimidinyl] -benzoesäure-isopropylester,
2-Cyano-4-fluor-5-[4-äthyl-3,6-dihydro-2,6-dioxo-1(2H)-pyrimidinyl]-benzoesäure-isopropylester,
2-Cyano-4-fluor-5-[4-äthyl-3,6-dihydro-3-methyl-2,6-dioxo-1(2H)-pyrimidinyl]-benzoesäure-isopropylester,
2-Cyano-4-fluor-5-[3,4-diäthyl-3,6-dihydro-2,6-dioxo-1(2H)-pyrimidinyl]-benzoesäure-isopropylester,
2-Cyano-4-fluor-5-[4-äthyl-3,6-dihydro-5-methyl-2,6-dioxo-1(2H)-pyrimidinyl]-benzoesäure-isopropylester,
2-Cyano-4-fluor-5-[4-äthyl-3,6-dihydro-3,5-dimethyl-2,6-dioxo-1(2H)-pyrimidinyl]-benzoesäure-isopropylester,
2-Cyano-4-fluor-5-[3,4-diäthyl-3,6-dihydro-5-methyl-2,6-dioxo-1(2H)-pyrimidinyl]-benzoesäure-isopropylester,
2-Cyano-4-fluor-5-[3,6-dihydro-4-isopropyl-2,6-dioxo-1(2H)-pyrimidinyl]-benzoesäure-isopropylester,
2-Cyano-4-fluor-5-[3,6-dihydro-4-isopropyl-3-methyl-2,6-dioxo-1(2H)-pyrimidinyl]-benzoesäure-isopropylester,
2-Cyano-4-fluor-5-[3-äthyl-3,6-dihydro-4-isopropyl-2,6-dioxo-1(2H)-pyrimidinyl]-benzoesäure-isopropylester,
2-Cyano-4-fluor-5-[3,6-dihydro-4-isopropyl-5-methyl-2,6-dioxo-1(2H)-pyrimidinyl]-benzoesäure-isopropylester,
2-Cyano-4-fluor-5-[3,6-dihydro-3,5-dimethyl-4-isopropyl-2,6-dioxo-1(2H)-pyrimidinyl]-benzoesäure-isopropylester,
2-Cyano-4-fluor-5-[3-äthyl-3,6-dihydro-4-isopropyl-5-methyl-2,6-dioxo-1(2H)-pyrimidinyl] -benzoesäure-isopropylester,
4-Chlor-2-cyano-5-(1,2,4,5,6,7-hexahydro-1-methyl-2,4-dioxo-3H-cyclopenta[d]-pyrimidin-3-yl)-benzoesäure-isopropylester,
4-Brom-2-cyano-5-(1,2,4,5,6,7-hexahydro-1-methyl-2,4-dioxo-3H-cyclopenta[d]-pyrimidin-3-yl)-benzoesäure-isopropylester,
2-Cyano-5-[3,6-dihydro-3-methyl-4-trifluormethyl-2,6-dioxo-1(2H)-pyrimidinyl]-benzoesäure-methylester und die entsprechenden Aethyl-, n-Propyl-, n-Butyl-, sek.Butyl-, Isobutyl-, tert.Butyl-, Methoxymethyl-, 1-Methoxyäthyl-, 2-Methoxyäthyl-, 1 -Methoxypropyl-, 1-Aethoxyäthyl-, 2-Methoxy- 1-methyläthyl-, Allyl-, 1-Methyl-2-propenyl-, 1-Aethyl-2-propenyl, 2-Butenyl-, Propargyl-, 2-Butinyl-, 1-Methyl-2-propinyl- und 1-Aethyl-2-propinylester dieser Verbindung.

2-Cyano-4-fluor-5-[3,6-dihydro-3-methyl-4-trifluormethyl-2,6-dioxo-1(2H)-pyrimidinyl]-benzoesäure-methylester und die entsprechenden Aethyl-, n-Propyl-, n-Butyl-, sek.Butyl-, Isobutyl-, tert.Butyl-, Methoxymethyl-, 1-Methoxyäthyl-, 2-Methoxyäthyl-, 1-Methoxy-propyl-, 1-Aethoxyäthyl-, 2-Methoxy- 1-methyläthyl-, Allyl-, 1 -Methyl-2-propenyl-, 1-Aethyl-2-propenyl, 2-Butenyl-, Propargyl-, 2-Butinyl-,1-Methyl-2-propinyl- und 1-Aethyl-2-propinylester dieser Verbindung.

Das erfindungsgemässe Verfahren zur Herstellung der Verbindungen der Formel I und ihrer Enoläther sowie Salze ist dadurch gekennzeichnet, dass man

a) Zwecks Herstellung derjenigen Verbindungen der Formel I, in denen $R_1$ Wasserstoff bedeutet und $R_2$ verschieden von Wasserstoff und $R_4$ verschieden von Chlor, Brom oder Jod sind, sowie gewünschtenfalls von Metallsalzen dieser Verbindungen, eine Verbindung der allgemeinen Formel

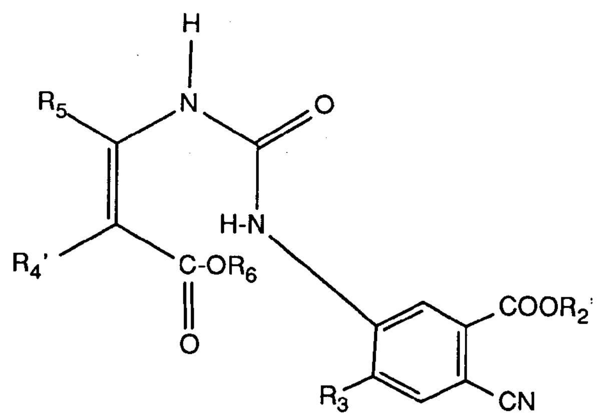

II

worin $R_3$ und $R_5$ die oben angegebenen Bedeutungen besitzen, $R_2'$die oben angegebene Bedeutung von $R_2$ mit Ausnahme von Wasserstoff besitzt, $R_4'$ Wasserstoff, Fluor oder $C_1$-$C_4$-Alkyl oder zusammen mit $R_5$ Tri- oder Tetramethylen bedeutet und $R_6$ nieder Alkyl, vorzugsweise $C_1$-$C_4$-Alkyl, bedeutet,

einer Cyclisierung unter basischen Bedingungen unterwirft und gewünschtenfalls eine allfällig erhaltene Metallsalzform des Uracilderivats durch Behandlung mit einer Säure in die entsprechende saure Form (R1 = Wasserstoff) überführt,

b) zwecks Herstellung derjenigen Verbindungen der Formel I, in denen $R_1$ Wasserstoff bedeutet und $R_2$ verschieden von Wasserstoff, $R_4$ verschieden von Chlor, Brom oder Jod und $R_5$ verschieden von $C_1$-$C_4$-Halogenalkyl sind, sowie gewünschtenfalls von Metallsalzen dieser Verbindungen, eine Verbindung der allgemeinen Formel

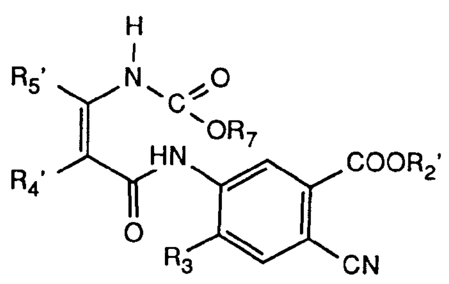

III

worin $R_2'$ und $R_3$ die oben angegebenen Bedeutungen besitzen, $R_4'$ Wasserstoff, Fluor oder $C_1$-$C_4$-Alkyl oder zusammen mit $R_5'$ Tri- oder Tetramethylen bedeutet, $R_5'$ $C_1$-$C_4$-Alkyl oder zusammen mit $R_4'$ Tri- oder Tetramethylen bedeutet, und $R_7$ nieder Alkyl, vorzugsweise $C_1$-$C_4$-Alkyl, bedeutet,

einer Cyclisierung unter basischen Bedingungen unterwirft und gewünschtenfalls ein allfällig erhaltenes Metallsalz des Uracilderivats der Formel I durch Behandlung mit einer Säure in die saure Form ($R_1$ = Wasserstoff) überführt.

c) zwecks Herstellung derjenigen Verbindungen der Formel I, in denen $R_1$ $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl bedeutet, ein Uracilderivat der allgemeinen Formel

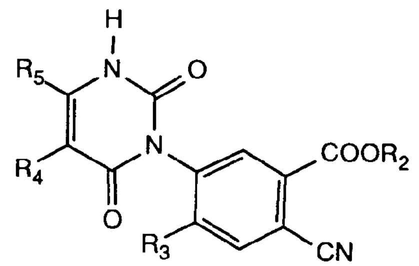

I'

worin $R_2$, $R_3$, $R_4$ und $R_5$ die oben angegebenen Bedeutungen besitzen, einer Alkylierung mit einem entsprechenden eine $C_1$-$C_4$-Alkyl-, $C_3$-$C_4$-Alkenyl-, $C_3$-$C_4$-Alkinyl-, $C_2$-$C_6$-Alkoxyalkyl- oder $C_1$-$C_4$-Halogenalkylgruppe enthaltenden Alkylierungsmittel unterwirft,

d) zwecks Herstellung sämtlicher Verbindungen der Formel I und der Enoläther ein Uracilderivat der allgemeinen Formel

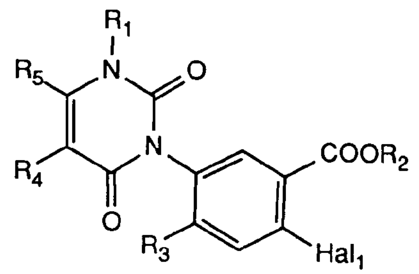

IV

worin $Hal_1$ Halogen, vorzugsweise Chlor oder Brom, bedeutet und $R_1$, $R_2$, $R_3$, $R_4$ und $R_5$ die oben angegebenen Bedeutungen besitzen, oder den entsprechenden Enoläther mit einem Metallcyanid behandelt,

e) zwecks Herstellung derjenigen Verbindungen der Formel I, in denen $R_2$ Wasserstoff bedeutet und $R_5$ verschieden von $C_1$-$C_4$-Halogenalkyl ist, und von deren Enoläthern einen Benzoesäureester der allgemeinen Formel

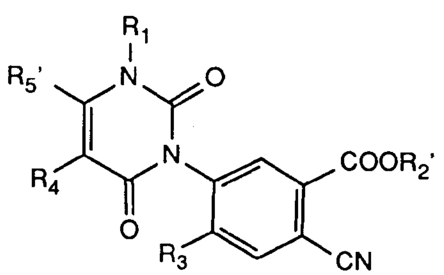

I''

worin $R_1$, $R_2'$, $R_3$, $R_4$ und $R_5'$die oben angegebenen Bedeutungen besitzen, zur entsprechenden Benzoesäure hydrolysiert,

f) zwecks Herstellung derjenigen Verbindungen der Formel I, in denen $R_1$ und $R_2$ jeweils verschieden von Wasserstoff und $R_5$ verschieden von $C_1$-$C_4$-Halogenalkyl sind, und der entsprechenden Enoläther eine Benzoesäure der allgemeinen Formel

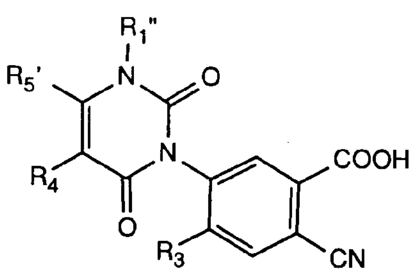

I'''

worin $R_1''$ $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl bedeutet und $R_3$, $R_4$ und $R_5$'die oben angegebenen Bedeutungen besitzen, oder den entsprechenden Enoläther, wobei die Benzoesäure oder deren Enoläther in Form eines reaktionsfähigen Derivats vorliegen kann, mit einer Hydroxyverbindung der allgemeinen Formel

$$HO\text{-}R_2'$$

worin $R_2'$die oben angegebene Bedeutung besitzt, oder mit einem reaktionsfähigen Derivat dieser Hydroxyverbindung verestert,

g) zwecks Herstellung derjenigen Verbindungen der Formel I, in denen $R_1$ und $R_2$ jeweils verschieden von Wasserstoff ist, und von deren Enoläthern einen Benzoesäureester der allgemeinen Formel

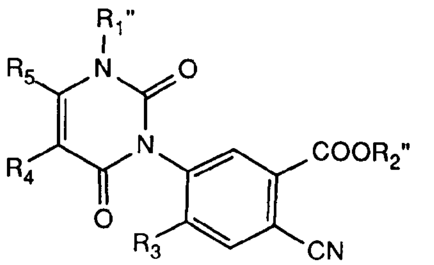

I''''

worin $R_1''$, $R_3$, $R_4$ und $R_5$ die oben angegebenen Bedeutungen besitzen und $R_2''$ $C_1$-$C_6$-Alkyl, $C_3$-$C_4$-Alkenyl, $C_3$-$C_4$-Alkinyl oder $C_2$-$C_6$-Alkoxyalkyl bedeutet, oder den entsprechenden Enoläther einer Umesterungsreaktion mit einer Hydroxyverbindung der oben angegebenen Formel V unterwirft, wobei das Reagens V höher siedend ist als das Alkanol, Alkenol bzw. Alkinol $R_2''$ OH,

h) zwecks Herstellung derjenigen Verbindungen der Formel I, in denen $R_4$ Chlor, Brom oder Jod bedeutet, und der entsprechenden Enoläther ein Uracilderivat der allgemeinen Formel

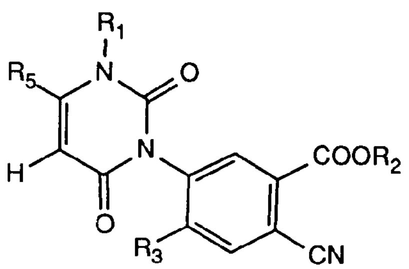

I'''''

worin $R_1$, $R_2$, $R_3$ und $R_5$ die oben angegebenen Bedeutungen besitzen, oder den entsprechenden Enoläther chloriert, bromiert bzw. jodiert,

i) zwecks Herstellung der Enoläther der Verbindungen der Formel I, ein Uracilderivat der allgemeinen Formel

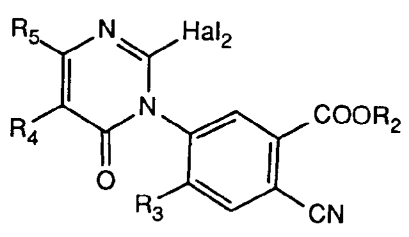

VI

worin $R_2$, $R_3$, $R_4$ und $R_5$ die oben angegebenen Bedeutungen besitzen und $Hal_2$ Chlor oder Brom bedeutet, mit einem Alkanol, Alkenol oder Alkinol $R_1'OH$ in Gegenewart einer organischen Base oder mit dem entsprechenden Alkoholat, Alkenolat bzw. Alkinolat der allgemeinen Formel

$$R_1'O^{\ominus}\ M^{\oplus} \qquad VII$$

worin $R_1'$ die oben angegebene Bedeutung besitzt und $M^{\oplus}$ ein Aequivalent eines Metallions bedeutet, behandelt, und gewünschtenfalls eine so erhaltene Verbindung der Formel I, in der $R_1$ und/oder $R_2$ Wasserstoff bedeutet, in ein Salz überführt.

Die Cyclisierung nach Verfahrensvariante a) oder b) kann zweckmässigerweise durchgeführt werden, indem man die Verbindung der Formel II bzw. III in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan oder Toluol, verwendet werden können; oder Wasser mit einer Base bei Temperaturen zwischen -78°C und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Falls als Lösungsmittel ein Alkanol verwendet wird, entspricht dieses Lösungsmittel zweckmässigerweise der jeweiligen Hydroxyverbindung $R_2'$-OH; dadurch werden unerwünschte konkurrierende Umesterungsreaktionen vermieden. Bei der Verwendung von Natriumhydrid als Base ist das Lösungsmittel vorzugsweise ein aliphatischer oder cyclischer Aether, Dimethylformamid oder Dimethylsulfoxid, wobei jedes dieser Lösungsmittel im Gemisch mit Toluol verwendet werden kann.

Nach Beendigung der Cyclisierung liegt das Produkt im Falle der Verwendung einer der obenerwähnten Basen oder dergleichen in Form des entsprechenden Alkalimetallsalzes vor. Dieses kann in an sich bekannter Weise isoliert und gereinigt werden, oder man kann das Gemisch ansäuren, um die jeweilige Verbindung der Formel I an sich zu isolieren. Zu diesem Zwecke verwendet man vorzugsweise eine Mineralsäure, wie Salzsäure, oder eine starke organische Säure, wie Essigsäure oder p-Toluolsulfonsäure.

Bei der Verfahrensvariante c) steht der Ausdruck "Alkylierung" für die Substitution des Wasserstoffatoms des $N_1$-Atoms des Uracilkerns mit einer $C_1$-$C_4$-Alkyl-, $C_3$-$C_4$-Alkenyl-, $C_3$-$C_4$-Alkinyl-, $C_2$-$C_6$-Alkoxyalkyl- oder $C_1$-$C_4$-Halogenalkylgruppe. Als Alkylierungsmittel wird zweckmässigerweise ein $C_1$-$C_4$-Alkyl-, $C_3$-$C_4$-Alkenyl-, $C_3$-$C_4$-Alkinyl- oder $C_2$-$C_6$-Alkoxyalkylhalogenid, insbesondere das diesbezügliche Chlorid oder Bromid, oder -sulfat bzw. ein mehrfach halogeniertes $C_1$-$C_4$-Alkan, wie beispielsweise Chlordifluormethan, oder ein mono- oder mehrfach halogeniertes Alken, wie beispielsweise Tetrafluoräthan, verwendet.

Die Alkylierung wird zweckmässigerweise in Gegenwart eines inerten, protischen organischen Lösungsmittels, wie eines niederen Alkanols, z.B. Aethanol, gegebenenfalls im Gemisch mit Wasser; eines inerten, aprotischen organichen Lösungsmittels, wie eines aliphatischen oder cyclischen Aethers, z.B. 1,2-Dimethoxyäthan, Tetrahydrofuran oder Dioxan; eines Ketons, z.B. Aceton oder Butan-2-on; oder eines inerten, aprotischen, polaren organischen Lösungsmittels, z.B. Dimethylformamid, Dimethylsulfoxid oder Acetonitril, sowie in Gegenwart einer Base, wie Natriumhydrid, eines Alkalimetallhydroxids, insbesondere Natrium- oder Kaliumhydroxid, eines Alkalimetallalkoholats, insbesondere Natriumalkoholat, oder eines Alkalimetallcarbonats oder -hydrogencarbonats, insbesondere Natriumcarbonat, Kaliumcarbonat, Natriumhydrogencarbonat oder Kaliumhydrogencarbonat, bei Temperaturen zwischen 0°C und der Rückflusstemperatur des Reaktionsgemisches, vorzugsweise bei Raumtemperatur oder, im Falle der Substitution des Wasserstoffatoms des $N_1$-Atoms mit einer $C_1$-$C_4$-Halogenalkylgruppe, vorzugsweise bei Temperaturen zwischen 50°C und 100°C, durchgeführt. In einer bevorzugten Ausführungsform wird das Uracilderivat der Formel I′ zunächst mit der Base, wie Natriumhydrid, -äthanolat oder -carbonat, im Lösungsmittel behandelt und nach einer kurzen Reaktionszeit mit dem Halogenid im gleichen Lösungsmittel versetzt. In einer weiteren Ausführungsform wird das Uracilderivat I′ zusammen mit einem Dialkylsulfat in Gegenwart eines Alkalimetallhydrogencarbonats, insbesondere Natrium- oder Kaliumhydrogencarbonat, im Lösungsmittel, z.B. Aceton, bei Rückflusstemperatur zur Reaktion gebracht. Die Reaktion ist in der Regel je nach verwendetem Lösungsmittel innert relativ kurzer Zeit oder nach wenigen Stunden beendet.

Bei der Verfahrensvariante d) handelt es sich um eine Austauschreaktion des Halogensubstituenten des Benzolkems. Dieses Halogenatom wird also durch die Cyanogruppe ersetzt, und zwar mittels des Metallcyanids. Letzteres ist insbesondere ein Uebergangsmetallcyanid, vorzugsweise Kuper(I)-cyanid. Die Umsetzung erfolgt zweckmässigerweise in Gegenwart eines aprotischen, polaren Lösungsmittels, wie eines Alkylnitrils, z.B. Aceto-, Propio- oder Butyronitril; eines Alkylharnstoffes, z.B. Tetramethylharnstoff; eines Dialkylamids, z.B. Dimethylformamid; eines Dialkylsufoxids, z.B. Dimethylsulfoxid; N-Methyl-2-pyrrolidon; 1,3-Dimethyl-imidazolidin-2-on; 1,3-Dimethyl-3,4,5,6-tetrahydro-2(IH)-pyrimidinon; oder Hexamethylphosphorsäuretriamid, bei erhöhten Temeraturen, und zwar zwischen 80°C und 200°C, vorzugsweise zwischen 150°C und 200°C. Im Ausgangsmaterial I″ bedeutet $R_4$ vorzugsweise Wasserstoff oder Fluor.

Die Hydrolyse des Benzoesäureesters I″ oder von dessen Enoläther nach Verfahrensvariante e) kann nach an sich bekannten Methoden durchgeführt werden, insbesondere unter Verwendung eines organischen Lösungsmittels in wässriger Lösung, wie wässriges Alkanol, z.B. Aethanol, oder ein aliphatischer oder cyclischer Aether, z.B. 1,2-Dimethoxyäthan, Tetrahydrofuran oder Dioxan, in wässriger Lösung, sowie einer anorganischen Base, wie Lithium-, Natrium- oder Kaliumhydroxid, oder eines Erdalkalimetallhydroxids, z.B. Magnesium- oder Calciumhydroxid, bei Temperaturen zwischen 0°C und 60°C, vorzugsweise bei Raumtemperatur.

Bei der Verfahrensvariante f) handelt es sich um eine Veresterung der Benzoesäure oder des Enoläthers bzw. eines reaktionsfähigen Derivats davon, die nach an sich bekannten Methoden durchgeführt werden kann. So wird beispielsweise ein Salz einer Benzoesäure der Formel I‴oder des entsprechenden Enoläthers mit einem Halogenid, insbesöndere Chlorid, Bromid oder Jodid, oder dem Sulfat, Mesylat oder Tosylat der Hydroxyverbindung V in einem inerten Verdünnungsmittel bei Temperaturen zwischen der Raumtemperatur und 100°C, z.B. bei der Rückflusstemperatur des Reaktionsgemisches, vorzugsweise im Temperaturbereich von 40°C bis 70°C, umgesetzt. Es kommen als Salze der Benzoesäure der Formel I‴ oder des entsprechenden Enoläthers insbesondere Alkalimetallsalze, z.B. das Natrium-, Kalium- oder Lithiumsalz, Erdalkalimetallsalze, z.B. das Magnesium-, Calcium- oder Bariumsalz, und Salze mit organischen Basen, wie tertiäre Amine, z.B. Triäthylamin, 1,5-Diaza-bicyclo[4,3,0]-non-5-en, 1,8-Diaza-bicyclo[5,4,0]-undec-7-en und 1,4-Diaza-bicyclo [2, 2,2]octan, in Frage, wobei die Alkalimetallsalze, insbesondere das Natriumsalz und das Kaliumsalz, bevorzugt sind. Die verwendbaren Verdünnungsmittel sind vorzugsweise inerte organische Lösungsmittel, wie niedere Alkanole, z.B. Aethanol, aliphatische und cyclische Aether, z.B. Diäthyläther, Tetrahydrofuran und Dioxan, Ketone, z.B. Aceton und 2-Butanon, Dimethylformamid, Dimethylsulfoxid, Acetonitril und Hexamethylphosphorsäuretriamid. Das Salz kann in situ hergestellt werden, indem man die Säure mit einer geeigneten anorganichen Base, z.B. einem Alkalimetall- oder Erdalkalimetallcarbonat, -hydrogencarbonat, -hydroxid oder -hydrid, bzw. organischen Base, zum Salz umsetzt und dies anschliessend im gleichen Reaktionsmedium mit dem zweiten Reaktionspartner reagieren lässt.

Im Falle der Verwendung eines Säurehalogenids der Benzoesäure der Formel I‴ oder des entsprechenden Enoläthers als reaktionsfähigen Derivats wird dies zweckmässigerweise mit der Hydroxyverbindung der Formel V in einem inerten organischen Lösungsmittel, wie einem aliphatischen oder cyclischen Aether, z.B. Diäthyläther, 1,2-Dimethoxyäthan, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid, Chloroform oder Tetrachlorkohlenstoff, bei Temperaturen von etwa -20°C bis 100°C, vorzugsweise von 0°C bis 50°C, umgesetzt. Zudem wird zweckmässigerweise in Gegenwart eines säurebindenden Mittels gearbeitet, wie einer organischen Base, z.B. Triäthylamin, Pyridin, 4-Dimethylaminopyridin, 1,5-Diaza-bicyclo[4,3,0]non-5-en, 1,8-Diaza-bicyclo[5,4,0]undec-7-en oder 1,4-Diazabicyclo[2,2,2]octan. Das Säurehalogenid ist vorzugsweise das Säurechlorid.

Als weitere in Frage kommende reaktionsfähige Derivate der Benzoesäure der Formel I‴ oder des entsprechenden Enoläthers seien der entsprechende O-Acyl-1,3-dicyclohexylisoharnstoff und das entsprechende N-Acylimidazol oder Säureanhydrid genannt. Solche Derivate können wie das Säurehalogenid mit den Hydroxyverbindungen der Formel V umgesetzt werden, um zu den gewünschten Benzoesäureestern zu gelangen. In diesen Fällen erübrigt sich jedoch die Verwendung eines säurebindenden Mittels.

Die Umsetzung nach Verfahrensvariante g) kann zweckmässigerweise durchgeführt werden, indem man den Benzoesäureester der Formel I⁗ oder dessen Enoläther in überschüssiger Hydroxyverbindung der Formel V in Gegenwart eines schwach basischen Katalysators, wie Natriumcyanid oder vorzugsweise Tetraisopropyl- oder Tetrapropylorthotitanat, erhitzt, und zwar vorzugsweise bei der Rückflusstemperatur des Reaktionsgemisches. Im Laufe der Reaktion wird der Rest $R_2''$ des Benzoesäureesters I⁗ durch die Gruppe $R_2'$ der Hydroxyverbindung V ersetzt, wobei das niedriger siedende Alkanol, Alkenol bzw. Alkinol $R_2''$ OH freigesetzt und aus dem Reaktionsgemisch entfernt wird.

Die Chlorierüng bzw. Bromierüng nach Verfahrensvariante h) wird zweckmässigerweise mittels elementaren Chlors oder Sulfurylchlorids bzw. elementaren Broms oder Sulfurylbromids durchgeführt, und zwar in Gegenwart eines inerten organischen Lösungsmittels, wie Essigsäure oder eines chlorierten aliphatischen Kohlenwasserstoffes, z.B. Methylenchlorid, Chloroform oder Tetrachlorkohlenstoff, und in einem Temperaturbereich von 0°C bis 60°C, vorzugsweise bei Raumtemperatur. Zudem kann die Umsetzung unter Zuhilfenahme eines säurebindenden Mittels erfolgen, zu welchem Zweck Natriumacetat und tertiäre Amine, wie Triäthylamin, Dimethylanilin und Pyridin, besonders bevorzugte säurebindende Mittel sind.

Die Jodierung nach dieser Verfahrensvariante erfolgt zweckmässigerweise unter Verwendung von elementarem Jod als Jodierungsmittel und von einer nieder siedenden aliphatischen Carbonsäure, wie Essigsäure, als Lösungsmittel und bei Temperaturen zwischen ca. 0°C und ca. 110°C, vorzugsweise bei Raumtemperatur. Zudem erweist es sich als zweckmässig, die Umsetzung in Gegenwart einer Säure, wie rauchender Salpetersäure, durchzuführen. Zur Beseitigung von überschüssigem Jod kann nach Beendung der Reaktion gesättigte wässrige Natriumhydrogensulfitlösung zugefügt werden.

Bei der Verfahrensvariante i) steht der Ausdruck "Metallion" insbesondere für ein Alkalimetallion, z.B. das Natrium- oder Kaliumion, oder ein Erdalkalimetallion, z.B. das Calcium- oder Magnesiumion. Das Natriumion ist das bevorzugte Metallion. Im Falle der Verwendung des Alkanols, Alkenols oder Alkinols $R_1'OH$ ist insbe-

sondere Pyridin die geeignete organische Base.

Die Umsetzung erfolgt zweckmässigerweise in einem Ueberschuss an dem entsprechenden Alkohol $R_1'$ OH als Verdünnungsmittel und bei Temperaturen zwischen 0°C und 50°C, vorzugsweise bei Raumtemperatur.

Sofern sie nicht unmittelbar durch die oben beschriebene unter basischen Bedingungen durchgeführte Cyclisierung herstellbar sind, können die gewünschten Salze der Verbindungen der Formel I, in denen $R_1$ Wasserstoff bedeutet, auch aus diesen Verbindungen I in an sich bekannter Weise hergestellt werden, wie beispielsweise durch Auflösen der Verbindung der Formel I in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen des Uracilderivats I in wässriger Natriumhydroxidlösung bei Raumtemperatur hergestellt, wobei äquivalente Mengen des Uracilderivats und des Natriumhydroxids verwendet werden. Das feste Salz kann dann durch Fällen mit einem geeigneten inerten Lösungsmittel oder durch Abdampfen des Lösungsmittels isoliert werden. Eine weitere Ausführungsform besteht darin, eine wässrige Lösung eines Alkalimetallsalzes des Uracilderivats I in eine wässrige Lösung eines Salzes, das ein anderes Metallion als ein Alkalimetallion aufweist, einzuführen, wobei das zweite Metallsalz des Uracilderivats hergestellt wird. Diese Ausführungsform dient im allgemeinen zur Herstellung von Uracil-Metallsalzen, die in Wasser unlöslich sind.

Die erhaltenen Verbindungen der Formel I, Enoläther sowie Salze können nach an sich bekannten Methoden isoliert und gereinigt werden. Ferner ist dem Fachmann geläufig, in welcher Reihenfolge allfällige Kombinationen der Verfahrensvarianten c) bis h) zweckmässig durchzuführen sind, um mögliche, unerwünschte konkurrierende Reaktionen zu vermeiden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsmaterialien der Formel II, die neu sind, können in an sich bekannter Weise hergestellt werden, z.B. gemäss den nachfolgenden Reaktionsschemata 1 [(Methodenaa), bb) und cc)]:

Reaktionsschemata 1

aa)

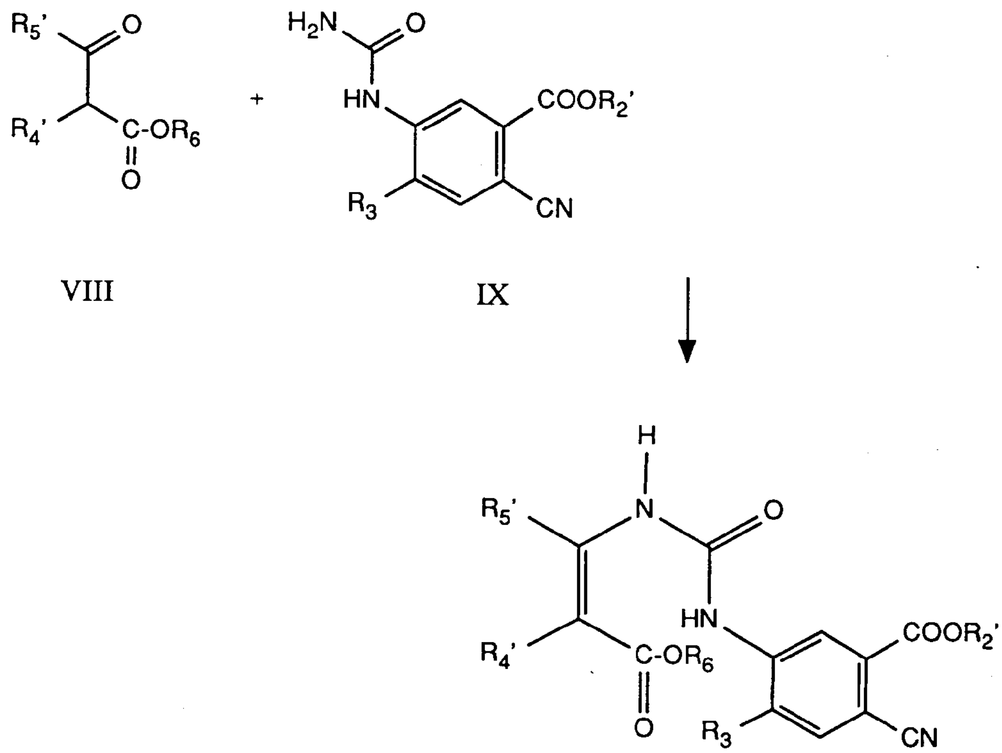

VIII

IX

II'

bb)

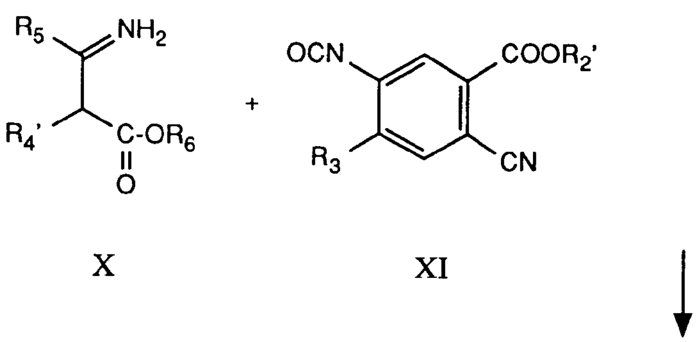

X

XI

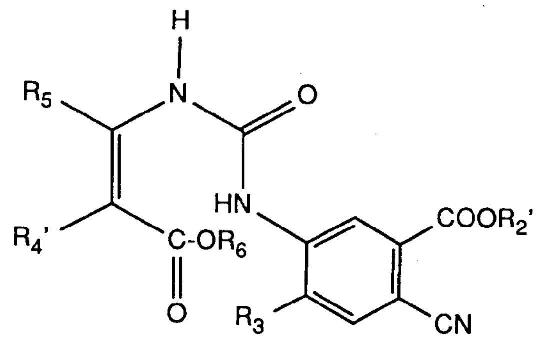

II

cc) 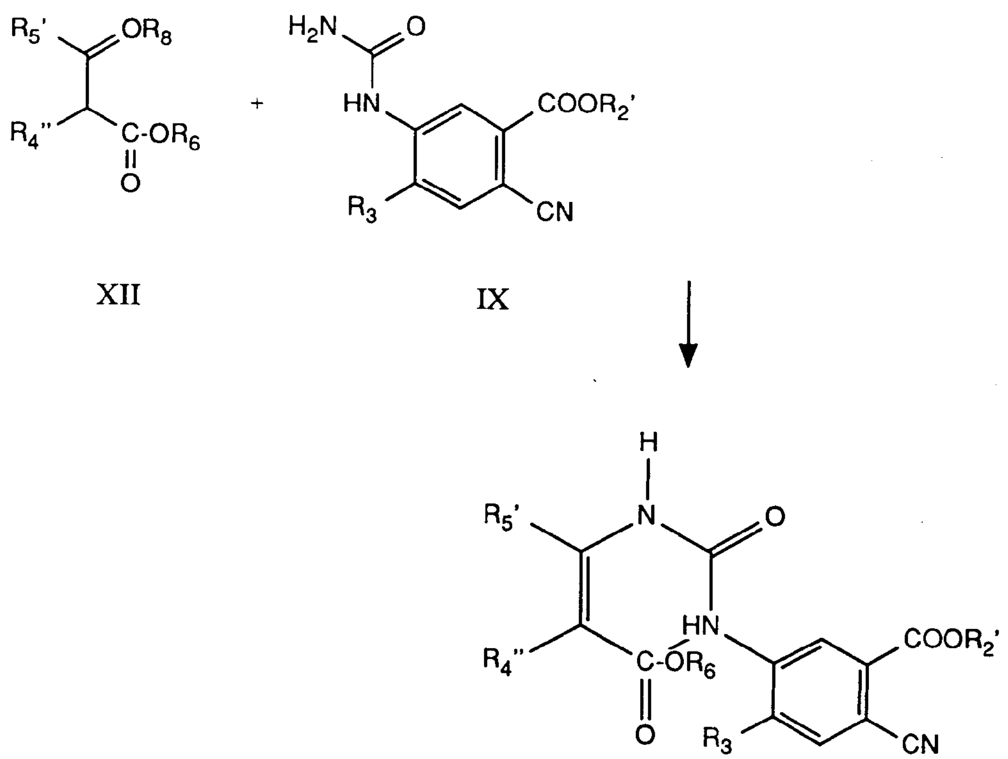

XII

IX

II"

In den obigen Reaktionsschemata besitzen $R_2'$, $R_3$, $R_4'$, $R_5$, $R_5'$und $R_5$ die oben angegebenen Bedeutungen; $R_4''$ bedeutet Wasserstoff, $C_1$-$C_4$-Alkyl oder zusammen mit $R_5'$ Tri- oder Tetramethylen; und $R_8$ bedeutet nieder Alkyl, vorzugsweise $C_1$-$C_4$-Alkyl.

Die Methode aa) wird zweckmässigerweise dadurch durchgeführt, dass man die Verbindungen der Formeln VIII und IX in einem im wesentlichen wasserfreien Verdünnungsmittel und in Gegenwart eines sauren Katalysators bei erhöhter Temperatur miteinandeir reagieren lässt. Als Verdünnungsmittel kommen insbesondere mit Wasser Azeotrope bildende organische Lösungsmittel, wie Aromaten, z.B. Benzol, Toluol und Xylole; halogenierte Kohlenwasserstoffe, wie Methylenchlorid, Chloroform, Tetrachlorkohlenstoff und Chlorbenzol; und aliphatische und cyclische Aether, wie 1,2-Dimethoxyäthan, Tetrahydrofuran und Dioxan, und als saure Katalysatoren insbesondere starke Mineralsäuren, wie Schwefelsäure und Salzsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70°C bis 120°C, vorzugsweise bei der Rückflusstemperatur des Reaktionsgemisches. Unter diesen Reaktionsbedingungen wird die erwünschte rasche Entfernung des in der Reaktion gebildeten

Wassers erzielt.

Die Umsetzung nach der Methode bb) erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols; oder eines halogenierten, aliphatischen Kohlenwasserstoffs, z.B. Methylenchlorid, Chloroform, Tetrachlorkohlenstoff oder 1,2-Dichloräthan, sowie gegebenenfalls in Gegenwart einer Base, insbesondere einer organischen tertiären Base, wie Triäthylamin oder Pyridin, wobei letzteres sowohl als Lösungsmittel wie auch als Base dienen kann, oder eines Metallhydrids, wie Natrium- oder Kaliumhydrid. Die Reaktionstemperaturen sind vorzugsweise im Bereich von etwa -80°C bis 50°C, wobei besonders bevorzugt bei Temperaturen zwischen -30°C und der Raumtemperatur gearbeitet wird.

Die Umsetzung nach der Methode cc) wird zweckmässigerweise in einem inerten, mit Wasser mischbaren, organischen Lösungsmittel, wie einem aliphatischen oder cyclischen Aether, z.B. 1,2-Dimethoxyäthan, Tetrahydrofuran oder Dioxan, oder einem niederen Alkanol, wie Aethanol, bei Temperaturen zwischen 50°C und 100°C, vorzugsweise bei der Rückflusstemperatur des Reaktionsgemisches, oder in einem aromatischen Lösungsmittel, wie Benzol, Toluol oder einem Xylol in Gegenwart eines sauren Katalysators, wie Salzsäure oder p-Toluolsulfonsäure, bei Temperaturen zwischen 50°C und 100°C, vorzugsweise 60°C bis 80°C, durchgeführt.

Die Ausgangsmaterialien der Formel II sind ebenfalls neu und können in an sich bekannter Weise hergestellt werden, z.B. gemäss den nachfolgenden Reaktionsschemata 2 [Methoden dd), ee) und ff)]:

Reaktionsschemata 2

dd)

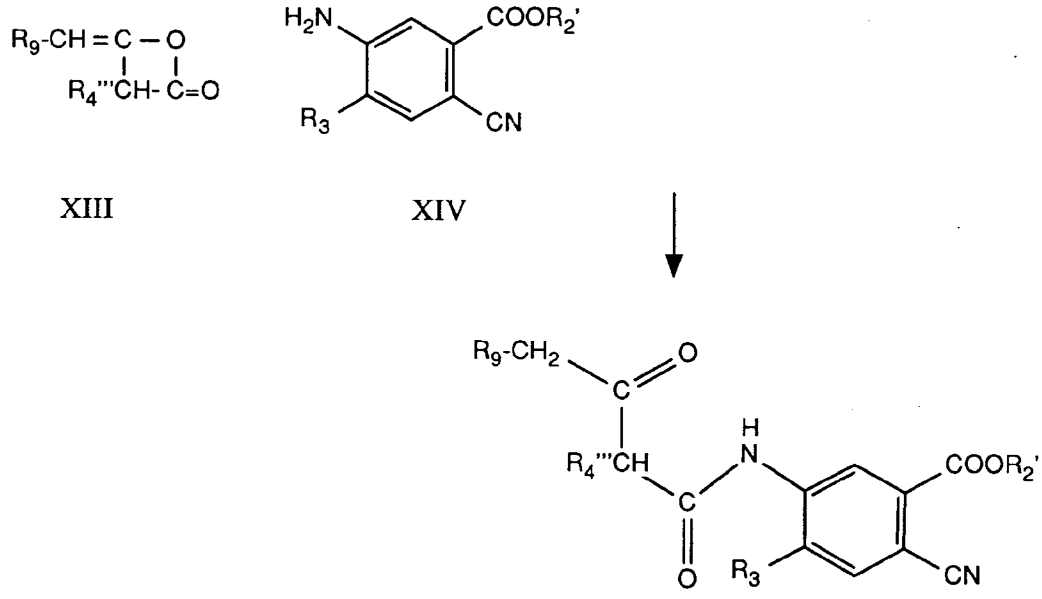

XIII

XIV

XV

ee)

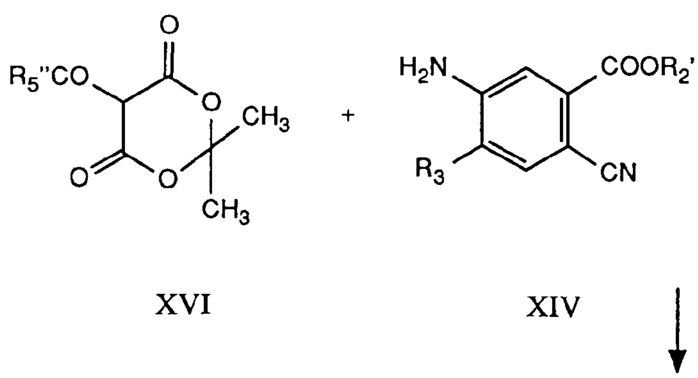

+

XVI

XIV

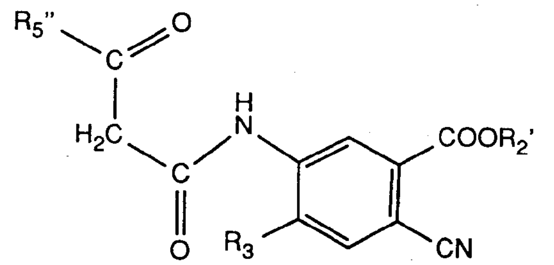

XVII

ff)

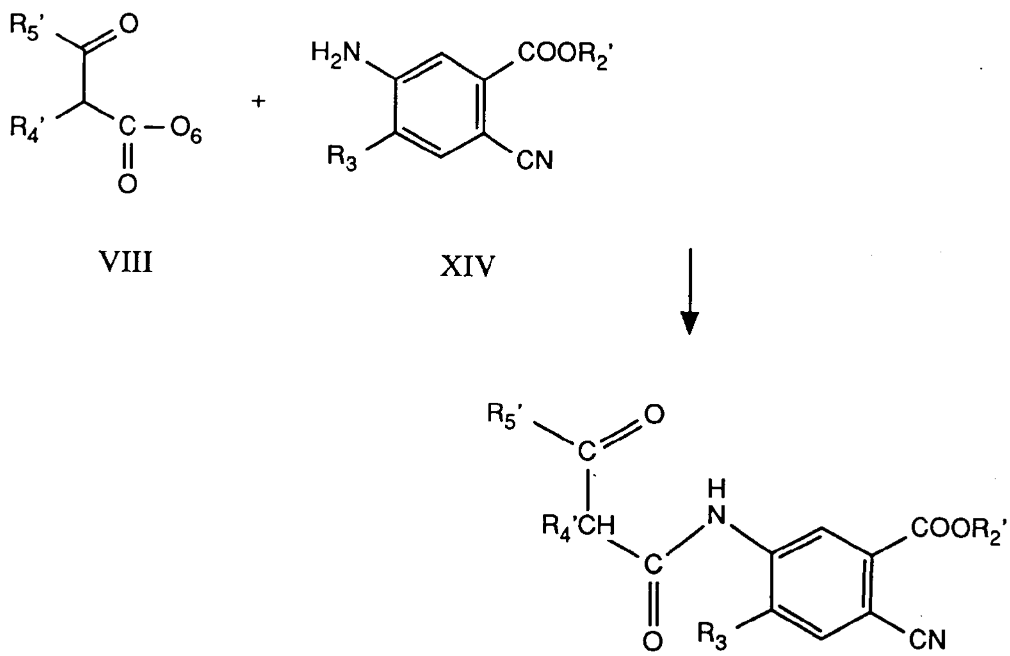

VIII + XIV

XVIII

XV/XVII/XVIII + $H_2NCOOR_7$ → III

XIX

In den obigen Reaktionsschemata besitzen $R_2'$, $R_3$, $R_4'$, $R_5'$, $R_6$ und X die oben angegebenen Bedeutungen; $R_4''$ bedeutet Wasserstoff oder $C_1$-$C_4$-Alkyl; $R_5''$ bedeutet $C_1$-$C_4$-Alkyl; und $R_9$ bedeutet Wasserstoff oder $C_1$-$C_3$-Alkyl.

Die Umsetzung des Amins der Formel XIV mit dem Diketen der Formel XIII nach der Methode dd) erfolgt zweckmässigerweise in einem wasserfreien inerten aprotischen Lösungsmittel, wie einem halogenierten Kohlenwasserstoff, z.B. Methylenchlorid, Chloroform, Tetrachlorkohlenstoff oder Chlorbenzol; einem aromatischen Kohlenwasserstoff, z.B. Benzol, Toluol oder einem Xylol, oder einem aliphatischen oder cyclischen Aether, z.B. Diäthyläther, 1,2-Dimethoxyäthan, Tetrahydrofuran oder Dioxan, in Gegenwart eines basischen Katalysators, wie 4-Pyrrolidino-pyridin, 4-Dimethylaminopyridin, 1,4-Diazabicyclo[2,2,2]octan, 1,5-Diazabicyclo[4,3,0]non-5-en, 1,8-Diazabicyclo[5,4,0]undec-7-en oder Diäthylamin. Da die Reaktion exothermisch ist, arbeitet man im allgemeinen in einem Temperaturbereich von - 10°C bis 50°C, vorzugsweise bei Raumtemperatur.

Die Umsetzung der Verbindungen der Formeln XVI und XIV miteinander nach der Methode ee) wird zweckmässigerweise in einem wasserfreien, inerten, aprotischen Lösungsmittel bei Temperaturen von etwa 70°C bis 140°C, vorzugsweise von 100°C bis 120°C, durchgeführt. Als derartige Lösungsmittel eignen sich insbesondere Aromaten, z.B. Benzol, Toluol und Xylole; halogenierte Kohlenwasserstoffe, z.B. Tetrachlorkohlenstoff, Trichloräthan, Tetrachloräthan und Chlorbenzol; und aliphatische und cyclische Aether, z.B. Dibutyläther, 1,2-Dimethoxyäthan, Tetrahydrofuran und Dioxan.

Bei der Umsetzung nach der Methode ff) handelt es sich um eine Aminolyse, die zweckmässigerweise in einem wasserfreien Lösungsmittel, oder ohne Lösungsmittel [siehe beispielsweise J. Soc. Dyes Col. 42, 81 (1926), Ber. 64, 970 (1931) und J.A.C.S.70, 2402 (1948)] bei erhöhter Temperatur erfolgt. Als Lösungsmittel kommen insbesondere inerte, aprotische Lösungsmittel, wie gegebenenfalls halogenierte Aromaten, z.B. Toluol, Xylole und Chlorbenzole, in Frage. Man arbeitet im allgemeinen in einem Temperaturbereich von etwa 130°C bis 160°C. Gegebenenfalls wird zudem in Gegenwart eines basischen Katalysators gearbeitet, z.B. eines höher siedenden Amins [siehe beispielsweise Helv. Chim. Acta 11, 779 (1928) und US-Patentschrift Nr. 2,416,738] oder Pyridin.

Die anschliessende Umsetzung der so hergestellten Verbindung der Formel XV, XVII bzw. XVIII mit dem Carbaminsäure-niederalkylester der Formel XIX erfolgt zweckmässigerweise in einem im wesentlichen wasserfreien Verdünnungsmittel und in Gegenwart eines sauren Katalysators bei erhöhter Temperatur. Als Verdünnungsmittel kommen insbesondere mit Wasser Azeotrope bildende organische Lösungsmittel, wie Aromaten, z.B. Benzol, Toluol und Xylole; cyclische Aliphate wie Cyclohexan; und halogenierte Kohlenwasserstoffe, wie Tetrachlorkohlenstoff und Chlorbenzol, und als saure Katalysatoren insbesondere starke Mineralsäuren, wie Schwefelsäure; organische Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70°C bis 150°C, vorzugsweise bei der Rückflusstemperatur des Reaktionsgemisches. Unter diesen Reaktionsbedingungen wird die erwünschte rasche Entfemung des in der Reaktion gebildeten Wassers erzielt.

Die Ausgangsmaterialien der Formel IV, die entsprechenden Enoläther und deren Herstellung sind grösstenteils in den europäischen Patentpublikationen Nm. 195 346 und 260 621 beschrieben. Diejenigen Ausgangsmaterialien IV und Enoläther, deren Herstellung nicht beschrieben ist, können analog den bekannten Ausgangsmaterialien hergestellt werden.

Die in der Verfahrensvariante i) verwendeten Ausgangsmaterialien der Formel VI können durch Halogenierung der entsprechenden Uracilderivate der oben angegebenen Formel I′ hergestellt werden. Bei dieser Halogenierung wird als Halogenierungsmittel insbesondere Thionylchlorid, Phosphorpentachlorid oder Phosphoroxychlorid bzw. Phosphorpentabromid oder Phosphorylbromid verwendet. Gegebenenfalls wird ein Gemisch von Phosphorpentachlorid und Phosphoroxychlorid bzw. von Phosphorpentabromid und Phosphorylbromid eingesetzt, wobei ein Ueberschuss an Phosphoroxychlorid bzw. Phosphorylbromid als Verdünnungsmittel dienen kann, Die Chlorierung bzw. Bromierung kann in Gegenwart eines inerten Verdünnungsmittels, insbesondere eines aprotischen organischen Lösungsmittels, wie eines aliphatischen oder aromatischen Kohlenwasserstoffes, z.B. n-Hexan, Benzol, Toluol oder eines Xylols; eines halogenierten aliphatischen Kohlenwasserstoffes, z.B. Methylenchlorid, Chloroform oder 1,2-Dichloräthan; eines halogenierten aromatischen Kohlenwasserstoffes, z.B. Chlorbenzol, oder eines tertiären Amins, z.B. N,N-Dimethylanilin, durchgeführt werden, jedoch ist dies im Falle der Verwendung von Phosphoroxychlorid bzw. Phosphorylbromid als Halogenierungsmittel nicht notwendig. Bei Verwendung von Thionylchlorid als Halogenierungsmittel erweist es sich als zweckmässig, eine katalytische Menge Dimethylformamid zuzusetzen. Die Reaktionstemperaturen liegen im allgemeinen zwischen 0°C und der Rückflusstemperatur des Reaktionsgemisches, vorzugsweise zwischen 80°C und 120°C.

Bei den als Ausgangsmaterialien in den Verfahrensvarianten c), e), f), g) und h) dienenden Uracilderivaten der Formel I′, I″, I‴, I⁗ bzw. I′′′′′ und Enoläther handelt es sich um Untergruppen von Verbindungen der Formel I und von deren Enoläthern. Die restlichen in den Verfahrensvarianten sowie in den Reaktionsschemata involvierten Ausgangsmaterialien bzw. Reagenzien sind entweder bekannt oder können nach an sich bekannten Methoden hergestellt werden.

Die Verbindungen der Formel I sowie ihre Enoläther und Salze (im folgenden insgesamt als "erfindungsgemässe Verbindungen" oder "Wirkstoffe" bezeichnet) sind geeignet, Pflanzenwachstum, insbesondere unerwünschtes Pflanzenwachstum, zu steuern, zu verhindern oder zu zerstören. Die erfindungsgemässen Verbindungen besitzen insbesondere herbizide Eigenschaften und eignen sich zur Bekämpfung von Unkräutern, einschliesslich Ungräser, beispielsweise Abutilon theophrasti, Amaranthus retroflexus, Agropyron repens, Alopecurus myosuroides, Avena fatua, Bromus inermis, Cyperus esculentus, Ipomoea purpurea, Poa annua, Sorghum halepense, Stellaria media, Cassia obtusifolia, Chenopodium album, Chrysanthemum segetum, Datura stramonium, Digitaria sanguinalis, Echinochloa crus-galli, Galium aparine, Matricaria chamomilla, Setaria faberii, Sinapis arvensis und Xanthium pennsylvanicum, in diversen Nutzpflanzenkulturen, beispielsweise

Raps-, Soya-, Baumwolle-, Reis-, Weizen- und Maiskulturen, insbesondere aber Baumwollkulturen. Zudem sind die Verbindungen sowohl Vorauflauf- als auch Nachauflauf-Herbizide. Des weiteren können die erfindungsgemässen Verbindungen zur Kontrolle von unerwünschtem Pflanzenwachstum, z.B. in Kartoffeln, Baumwolle, Sonnenblumen, Saatgemüse und Wasserunkräutern, verwendet werden. Sie können beispielsweise als Abbrennmittel zur Erleichterumg der Ernte bei Kartoffeln und Baumwolle verwendet werden.

In der Praxis genügt üblicherweise eine Konzentration von 0,5 g bis 6,0 kg erfindungsgemässe Verbindung/ha, vorzugsweise 1 g bis 200 g erfindungsgemässe Verbindung/ha, um den gewünschten herbiziden Effekt zu erzielen.

Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Das erfindungsgemässe Unkrautbekämpfungsmittel ist dadurch gekennzeichnet, dass es eine wirksame Menge mindestens einer Verbindung der Formel I, wie oben definiert, oder eines Enoläthers oder Salzes davon sowie Formulierungshilfsstoffe enthält. Das Mittel enthält zweckmässigerweise zumindest einen der folgenden Formulierungshilfsstoffe; feste Trägerstoffe; Lösungs- bzw. Dispersionsmittel; Tenside (Netzmittel und Emulgatoren); Dispergiermittel (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen emulgierbare Konzentrate, Pasten und dergleichen.

Die Verbindungen der Formel I und ihre Enoläther sind im allgemeinen wasserunlöslich, die Salze hingegen, insbesondere die Alkalimetallsalze und Ammoniumsalze, im allgemeinen wasserlöslich, und können nach den für wasserunlösliche bzw. wasserlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netzmittel oder Emulgatoren und/oder von Dispergiermitteln, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdöfraktionen; Alkohole, wie Butanol und Glykol, sowie deren Aether und Ester, Ketone, wie Aceton, Methyläthylketon, Methylisobutylketon und Cyclohexanon; und stark polare Lösungs-bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere AerosolTreibgase, wie Kohlenwasserstoffe, z.B. Propan und Isobutan, und Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netzmittel und Emulgatoren) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Alkylsulfonate und fettaromarische Sulfonate, wie Alkylbenzolsulfonate, z.B. Calcium-dodecylbenzolsulfonat, und Butylnaphtalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergiermittel, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butyl-hydroxytoluol; UV-Absorber, z.B. substituierte Benzophenone, Diphenylacrylonitrilsäureester und Zimtsäureester, und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,01 und 95 Gewichtsprozent, vorzugsweise zwischen 0,5 und 75 Gewichtsprozent einer bzw. mehrerer erfindungsgemässer Verbindungen als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10 und 20 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,01 bis 10 Gewichtsprozent, insbesondere ca. 0,005 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine erfindungsgemässe Verbindung, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfemen. Durch Zusatz von Tensiden bzw. Dispergiermitteln kann man solche pulverförmige Mittel mit Wasser leicht benetzbar machen, so dass sie in wässriger Suspension, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. mit einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

Beispiel 1:

Ein Gemisch von 50,0 g 2-Chlor-5-[3,6-dihydro-3,4-dimethyl-2,6-dioxo-1(2H)-pyrimidinyl]-4-fluorbenzoesäure-isopropylester und 25,4 g Kupfer(I)cyanid in 225 ml Dimethyl-3,4,5,6-tetrahydro-2(1H)-pymidinon wird unter Stickstoff 2,5 Stunden auf 195°C erhitzt. Anschliessend wird das Gemisch auf Raumtemperatur abgekühlt und mit 2l Essigsäure-äthylester versetzt. Dann giesst man das Gemisch auf 1,3l Wasser und 300 ml 32%ige

Salzsäure und rührt das Ganze 45 Minuten bei Raumtemperatur, bis sich zwei klare Schichten gebildet haben. Die organische Phase wird abgetrennt, zweimal mit je 200 ml Wasser gewaschen und über wasserfreiem Natriumsulfat getrocknet. Durch Eindampfen der organischen Lösung erhält man den kristallinen 2-Cyano-5-[3,6-dihydro-3,4-dimethyl-2,6-dioxo-1(2H)-pyrimidinyl]-4-fluorbenzoesäure-isopropylester, Smp. 188-200°C.

Beispiele 2-10:

Analog dem in Beispiel 1 beschriebenen Verfahren erhält man durch Behandlung des entsprechenden Uracilderivats der Formel IV mit Kupfer(I)cyanid die in der nachstehenden Tabelle 1 aufgeführten Verbindungen der Formel I:

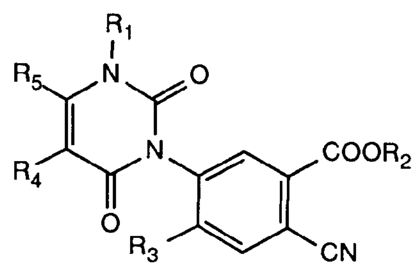

| Beispiel | $R_1$ | $R_2$ | $R_3$ | $R_4$ | $R_5$ | Physikalische Daten |
|---|---|---|---|---|---|---|
| 2 | $CH_3$ | $CH(CH_3)_2$ | H | H | $CF_3$ | Smp. 125-127°C |
| 3 | $CH_3$ | $CH(CH_3)_2$ | F | H | $CF_3$ | Smp. 130-132°C |
| 4 | $CH_3$ | $CH(CH_3)_2$ | F | H | $C_2F_5$ | Smp. 116-118°C |
| 5 | $CH_3$ | $CH(CH_3)_2$ | F | $-(CH_2)_4-$ | | Smp. 170-172°C |
| 6 | $CH_3$ | $CH(CH_3)_2$ | F | $-(CH_2)_3-$ | | Smp. 164-166°C |
| 7 | $CH_3$ | $CH(CH_3)_2$ | F | F | $CF_3$ | Smp. 133-136°C |
| 8 | $CH_3$ | $CH_3$ | H | H | $CF_3$ | Smp. 167-168°C |
| 9 | $CHF_2$ | $CH(CH_3)_2$ | F | $-(CH_2)_4-$ | | Smp. 142-145°C |
| 10 | $CHF_2$ | $CH(CH_3)_2$ | F | H | $CH_3$ | Smp. 146-148°C |

Beispiel 11

Eine Lösung von 30,5 g 2-Cyano-5-[3,6-dihydro-3,4-dimethyl-2,6-dioxo -1(2H)-pyrimidinyl] -4-fluorbenzoesäure-isopropylester (siehe Beispiel 1 ) in 150 ml Methanol wird mit 4,0 g Natriumhydroxid in 50 ml Wasser 15 Minuten bei 1-3°C gehalten und anschliessend 23 Stunden nachgerührt. Die Lösung wird unter vermindertem Druck weitgehend eingedampft und der Rückstand mit 2N Salzsäure auf den pH-Wert 2 gebracht. Der resultierende Niederschlag wird abgenutscht und mit n-Hexan nachgewaschen. Man erhält auf diese Weise die -Cyano-5-[3,6-dihydro-3,4-dimethyl-2,6-dioxo-1(2H)-pyrimidinyl]-4-fluorbenzoesäure, Smp. 232°C (unter Zersetzung).

Beispiel 12

Eine Lösung von 2-Cyano-5-[3,6-dihydro-3,4-dimethyl-2,6-dioxo - 1(2H)-pyrimidinyl]-4-fluorbenzoesäure (siehe Beispiel 9) in 30 ml absolutem Dimethylformamid wird mit 0,29 g einer 55%igen Natriumhydrid-Dispersion 2 Stunden bei Raumtemperatur gerührt. Anschliessend wird eine Lösung von 1,1 g Propargylbromid in

10 ml absolutem Dimethylformamid innert 10 Minuten zugetropft, und das Reaktionsgemisch wird 3 Stunden nachgerührt.

Man löst dann das Gemisch in 100 ml Essigsäure-äthylester, wäscht die Lösung gründlich mit Wasser, trocknet die organische Phase über wasserfreiem Natriumsulfat und dampft sie unter vermindertem Druck zur Trockene ein. Der Rückstand wird an einer Kieselgel-Säule unter Verwendung von n-Hexan/Essigsäure-äthylester (3:7) als Laufmittel chromatographisch gereinigt. Auf diese Weise erhält man den 2-Cyano-5-[3,6-dihydro-3,4-dimethyl-2,6-dioxo - 1(2H)-pyrimidinyl]-4-fluorbenzoesäure-propargylester, Smp. 174-177°C.

Beispiele 13-15

Analog dem in Beispiel 12 beschriebenen Verfahren erhält man durch Veresterung von 2-Cyano-5-[3,6-dihydro-3,4-dimethyl-2,6-dioxo - 1(2H)-pyrimidinyl]-4-fluorbenzoesäure (siehe Beispiel 11 ) mit Allylbromid, Methyljodid bzw. 2-Methoxy-äthylbromid die in der nachstehenden Tabelle 2 aufgeführten Verbindungen der Formel I:

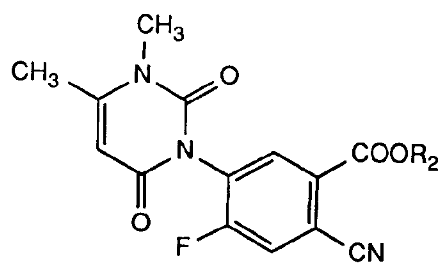

Tabelle 2

| Beispiel | $R_2$ | Physikalische Daten |
|---|---|---|
| 13 | Allyl | Smp. 143-148°C |
| 14 | Methyl | Smp. 244-246°C |
| 15 | 2-Methoxyäthyl | Smp. 126-130°C |

Beispiel 16

Ein Gemisch von 3,0 g feinpulverisierter 2-Cyano-5-[3,6-dihydro-3,4-dimethyl-2,6-dioxo - 1(2H)-pyrimidinyl]-4-fluorbenzoesäure, 30 ml frisch destilliertem Thionylchlorid und 3 Tropfen Dimethylformamid wird unter Rühren 1 Stunde auf Rückflusstemperatur erhitzt, wonach sich eine klare Lösung gebildet hat. Das Gemisch wird dann zur Trockene eingedampft. Auf diese Weise erhält man das 2-Cyano-5-[3,6-dihydro-3,4-dimethyl-2,6-dioxo - 1(2H)-pyrimidinyl]-4-fluorbenzoesäurechlorid, Smp. 160°C, das ungereinigt als Ausgangsmaterial in der nächsten Reaktionsstufever wendet wird.

Zu einer Lösung von 3,8 g 2-Cyano-5-[3,6-dihydro-3,4-dimethyl-2,6-dioxo -1(2H)-pyrimidinyl] -4-fluorbenzoesäurechlorid in 130 ml Tetrahydrofuran wird bei Raumtemperatur eine Lösung von 1,5 g 1,2,3,6-Tetrahydrobenzylalkohol und 0,015 g 4-Dimethylaminopyridin in 2,5 g Pyridin getropft. Nach 12-stündigern Erhitzen des Reaktionsgemisches bei 60°C wird die resultierende Suspension unter vermindertem Druck weitgehend eingedampft und der Rückstand in Essigsäure-äthylester gelöst.

Die organische Lösung wird gründlich mit Wasser gewaschen, die organische Phase über wasserfreiem Natriumsulfat getrocknet und unter vermindertem Druck zur Trockene eingedampft, und der Rückstand an einer Kieselgel-Säule unter Verwendung von n-Hexan/Essigsäure-äthylester (3:7) als Laufmittel chromatographisch gereinigt. Man erhält auf diese Weise den 2-Cyano-5-[3,6-dihydro-3,4-dimethyl-2,6-dioxo - 1 (2H)-pyrimidinyl]-4-fluorbenzoesäure - 1,2,3,6-tetrahydrobenzylester, Massenspektrum (m/e): 397(15)$M^+$.

Biologische Beispiele

Beispiel B1: Pre-emergente Herbizid-Wirkung

Im Gewächshaus wird unmittelbar nach der Einsaat der Versuchspflanzen (eine Anzahl Unkräuter, sowohl monokotyle wie dikotyle) in Saatschalen die Erdoberfläche mit einer wässrigen Spritzbrühe entsprechend einer Aufwandmenge von 3 kg Wirksubstanz/Hektar behandelt.

Die Test-Substanzen werden vorzugsweise als emulgierbares Konzentrat (EC) formuliert und unmittelbar vor der Applikation in Wasser auf die gewünschte Konzentration verdünnt. Unlösliche Substanzen werden mittels Kaolin als inertem Träger als benetzbares Pulver (WP) formuliert. Dieses wird unmittelbar vor der Applikation in Wasser suspendiert.

Die Dosierungen in g Aktivsubstanz/ha beziehen sich auf die Erdoberfläche in den Behältern, soweit nicht anders angegeben. Das Sprühvolumen beträgt 1000 l/ha (entsprechend 100 ml/$m^2$).

Die Samen der Pflanzen werden in Kunststoff-Pflanztöpfe verschiedener Größe mit hitzesterilisierter (gedämpfter) Erde gesät (Landerde 2.6% Torf, 20% Ton, 30% Schluff, 47% Sand). Die Pflanzen werden im Gewächshaus bei mittlerer Temperatur (17 - 25°C im Winter, 18 - 35°C im Sommer) gehalten (Luftfeuchte 30 - 90%). Die Länge der Photoperiode beträgt 13 bis 16 Stunden/Tag, falls erforderlich, wird künstliches Licht (15000 bis 18000 Lux) zugeschaltet. Die künstliche Beleuchtung wird auch bei unzureichender Tageslicht-Intensität automatisch aktiviert.

Nach 3 Wochen wird die Herbizidwirkung mit einem dreistufigen linearen Boniturschema (Necrose, Chlorose, Reduktion, Deformation) im Vergleich zu einer unbehandelten Kontrollgruppe bewertet (2 = 80-100% Schädigung, 1 = 30-79% Schädigung, 0 = 0-29% Schädigung).

Tabelle B1: preemergente Herbizidwirkung:

| Beispiel | SORG | ECHI | AVEN | ALOP | CHEN | STEL | ABUTU | DATA | MATRS | CASS |
|---|---|---|---|---|---|---|---|---|---|---|
| 01 | 2 | 2 | 2 | 2 | 2 | 2 | 2 | 2 | 2 | 2 |
| 02 | 2 | 2 | 2 | 2 | 2 | 2 | 2 | 2 | 2 | 2 |
| 03 | 2 | 2 | 2 | 2 | 2 | 2 | 2 | 2 | 2 | 2 |
| 04 | 2 | 2 | 2 | 2 | 2 | 2 | 2 | 2 | 2 | 2 |
| 05 | 2 | 2 | 2 | 2 | 2 | 2 | 2 | 2 | 2 | 2 |
| 06 | 2 | 2 | 2 | 2 | 2 | 2 | 2 | 2 | 2 | 2 |
| 07 | 2 | 2 | 2 | 2 | 2 | 2 | 2 | 2 | 2 | 2 |
| 08 | 2 | 2 | 2 | 2 | 2 | 2 | 2 | 2 | 2 | 2 |
| 09 | 2 | 2 | 2 | 2 | 2 | 2 | 2 | 2 | 2 | 2 |
| 10 | 2 | 2 | 2 | 2 | 2 | 2 | 2 | 2 | 2 | 2 |
| 12 | 2 | 2 | 2 | 2 | 2 | 2 | 2 | 2 | 2 | 2 |
| 13 | 2 | 2 | 2 | 2 | 2 | 2 | 2 | 2 | 2 | 2 |
| 14 | 2 | 2 | 2 | 2 | 2 | 2 | 2 | 2 | 2 | 2 |
| 15 | 2 | 2 | 2 | 2 | 2 | 2 | 2 | 2 | 2 | 2 |

In der Tabelle bedeuten :

| | |
|---|---|
| SORG | Sorghum halopense |
| ECHI | Echinochloa crus-galli |
| AVEN | Avena fatua |
| ALOP | Alopecurus myosuroides |
| CHEN | Chenopodium album |
| STEL | Stellaria media |
| ABUT | Abutilon theophrasti |
| MATR | Matricaria chamomilla |
| CASS | Cassia media |

Beispiel B2: Post-emergente Herbizid-Wirkung (Kontaktherbizid)

Eine Anzahl Unkräuter, sowohl monokotyle wie dikotyle, wurden nach dem Auflaufen (im 2- bis 6-Blattstadium) mit einer wäßrigen Wirkstoffdispersion in einer Dosierung von 3 kg Wirksubstanz pro Hektar behandelt.

Die Test-Substanzen werden vorzugsweise als emulgierbares Konzentrat (EC) formuliert und unmittelbar vor der Applikation in Wasser auf die gewünschte Konzentration verdünnt. Unlösliche Substanzen werden mittels Kaolin als inertem Träger als benetzbares Pulver (WP) formuliert. Dieses wird unmittelbar vor der Applikation in Wasser suspendiert

Die Dosierungen in g Aktivsubstanz/ha beziehen sich auf die Erdoberfläche in den Behältern, soweit nicht anders angegeben. Das Sprühvolumen beträgt 500 l/ha.

Die Samen der Pflanzen werden in Plastik-Pflanztöpfe verschiedener Größe mit hitzesterilisierter (gedämpfter) Erde gesät ('Optima'Erde 80% Torf, 20% Löß). Die Pflanzen werden im Gewächshaus bei mittlerer Temperatur ( 17 - 25 °C im Winter, 18 - 35°C im Sommer) gehalten (Luftfeuchte 30 - 90%). Die Länge der Photoperiode beträgt 13 bis 16 Stunden/Tag, falls erforderlich, wird künstliches Licht (15000 bis 18000 Lux) zugeschaltet. Die künstliche Beleuchtung wird auch bei unzureichender Tageslicht-Intensität automatisch aktiviert.

Nach 3 Wochen wird die Herbizidwirkung mit einem elfstufigen linearen Boniturschema (Necrose, Chlorose, Reduktion, Deformation) im Vergleich zu einer unbehandelten Kontrollgruppe bewertet (2 = 80-100% Schädigung, 1 = 30-79% Schädigung, 0 = 0-29% Schädigung).

Tabelle B2: postemergente Herbizidwirkung:

| Beispiel | SORG | ECHI | AVEN | ALOP | CHEN | STEL | ABUTU | DAT | MATR | CASS |
|---|---|---|---|---|---|---|---|---|---|---|
| 01 | 2 | 2 | 1 | 1 | 2 | 1 | 2 | 2 | 2 | 2 |
| 02 | 2 | 2 | 2 | 2 | 2 | 2 | 2 | 2 | 2 | 2 |
| 03 | 2 | 2 | 2 | 2 | 2 | 2 | 2 | 2 | 2 | 2 |
| 04 | 2 | 2 | 2 | 2 | 2 | 2 | 2 | 2 | 2 | 2 |
| 05 | 2 | 2 | 2 | 1 | 2 | 2 | 2 | 2 | 2 | 2 |
| 06 | 2 | 2 | 2 | 1 | 2 | 2 | 2 | 2 | 2 | 2 |
| 07 | 2 | 2 | 2 | 2 | 2 | .2 | 2 | 2 | 2 | 2 |
| 08 | 2 | 2 | 2 | 2 | 2 | 2 | 2 | 2 | 2 | 2 |
| 10 | 2 | 2 | 2 | 2 | 2 | 2 | 2 | 2 | 2 | 2 |
| 13 | 2 | 2 | 2 | 1 | 2 | 1 | 2 | 2 | 2 | 1 |

Formulierungsbeispiele für Wirkstoffe der Formel I
(% = Gewichtsprozent)

| 1. Spritzpulver | a) | b) | c) |
|---|---|---|---|
| Wirkstoff gemäß Beispiel 1-15 | 20 % | 50 % | 0,5 % |
| Na-Ligninsulfonat | 5 % | 5 % | 5 |
| Na-Laurylsulfat | 3 % | - | - % |
| Na-Diisobutylnaphthalinsulfonat | - | 6 % | 6 % |
| Octylphenolpolyethylenglykolether (7-8 Mol AeO) | - | 2 % | 2 |
| Hochdisperse Kieselsäure | 5 % | 27 % | 27 % |
| Kaolin | 67 % | - % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrate | a) | b) |
|---|---|---|
| Wirkstoff gemäß Beispiel 1-15 | 10% | 1 % |
| Ca-Dodecylbenzolsulfonat | 3 % | 3 % |
| Octylphenolpolyethylenglykolether (4-5 Mol AeO) | 3 % | 3 % |
| Ricinusöl-polyethylenglykolether (36 Mol AeO) | 4 % | 4 |
| Cyclohexanon | 30 | 10 % |
| Xylolgemisch | 50 % | 79 % |

Aus solchen Konzentraten können durch Verdünnen mit Wasser Emulsionen jeder gewünschten Konzentration hergestellt werden.

| 3. Stäubemittel | a) | b) |
|---|---|---|
| Wirkstoff gemäß Beispiel 1-15 | 0,1 % | 1 % |
| Talkum | 99,9 % | - |
| Kaolin | - | 99 % |

Durch inniges Vermischen der Trägerstoffe mit dem Wirkstoff erhält man gebrauchsfertige Stäubemittel.

| 4. Extruder-Granulat | a) | b) |
|---|---|---|
| Wirkstoff gemäß Beispiel 1-15 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschließend im Luftstrom getrocknet.

| 5. Umhüllungs-Granulat | |
|---|---|
| Wirkstoff gemäß Beispiel 1-15 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 6. Suspensions-Konzentrat | a) | b) |
|---|---|---|
| Wirkstoff gemäß Beispiel 1-15 | 5 % | 40 % |
| Ethylenglykol | 10 % | 10 % |
| Nonylphenolpolyethylenglykolether (15 Mol AeO) | 1 % | 6 % |
| Na-Ligninsulfonat | 5 % | 10 % |
| Carboxymethylcellulose | 1 % | 1 % |
| 37 %ige wäßrige Formaldehyd-Lösung | 0,2 % | 0,2 % |
| Silikonöl in Form einer 75%-igen wäßrigen Emulsion | 0,8 % | 0,8 % |
| Wasser | 77 % | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

7. Salzlösung

| | |
|---|---|
| Wirkstoff gemäß Beispiel 1-15 | 5 % |
| Isopropylamin | 1 % |
| Octylphenolpolyethylenglycol-ether (78 Mol AeO) | 3 % |
| Wasser | 91% |

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

**Patentansprüche**

1. Verbindungen der allgemeinen Formel

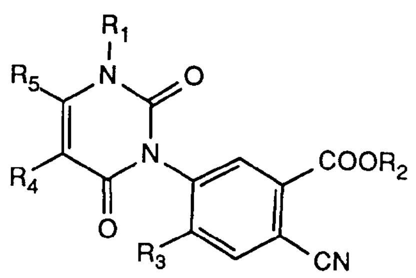

I

worin
$R_1$ Wasserstoff, $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl,
$R_2$ Wasserstoff, $C_1$-$C_6$-Alkyl, $C_2$-$C_6$-Alkoxyalkyl, gegebenenfalls mit 1 bis 3 $C_1$-$C_3$-Alkylgruppen ringsubstituiertes $C_4$-$C_7$-Cycloalkenyl-$C_1$-$C_4$-alkyl, $C_3$-$C_4$-Alkenyl, gegebenenfalls mit 1 bis 3 $C_1$-$C_3$-Alkylgruppen ringsubstituiertes $C_1$-$C_7$-Cycloalkenyl-$C_3$-$C_5$-alkenyl, Aryl-$C_3$-$C_5$-alkenyl, $C_3$-$C_4$-Alkinyl, gegebenenfalls mit 1 bis 3 $C_1$-$C_3$-Alkylgruppen ringsubstituiertes $C_4$-$C_7$-Cycloalkenyl-$C_3$-$C_5$-alkinyl oder Aryl-$C_3$-$C_5$-alkinyl,
$R_3$ Wasserstoff oder Halogen,
$R_4$ Wasserstoff, Halogen oder $C_1$-$C_4$-Alkyl und
$R_5$ $C_1$-$C_4$-Alkyl oder $C_1$-$C_4$-Halogenalkyl, oder
$R_4$ und $R_5$ zusammen Tri- oder Tetramethylen bedeuten, mit der Massgabe, dass, falls $R_5$ für $C_1$-$C_4$-Halogenalkyl steht, $R_1$ verschieden von $C_1$-$C_4$-Halogenalkyl und $R_2$ verschieden von Wasserstoff ist,
und die entsprechenden Enoläther derjenigen Verbindungen der Formel 1, in denen $R_1$ voll Wasserstoff oder $C_1$-$C_4$-Halogenalkyl verschieden ist, sowie Salze derjenigen Verbindungen der Formel I, in denen $R_1$ und/oder $R_2$ Wasserstoff bedeutet.

2. Verbindungen nach Anspruch 1, worin $R_1$ $C_1$-$C_4$-Alkyl bedeutet.

3. Verbindungen nach Anspruch 1 oder 2, worin $R_2$ $C_1$-$C_6$-Alkyl, $C_2$-$C_6$-Alkoxyalkyl, $C_3$-$C_4$-Alkenyl oder $C_3$-$C_4$-Alkinyl bedeutet.

4. Verbindungen nach einem der Ansprüche 1 bis 3, worin $R_3$ Wasserstoff oder Fluor bedeutet.

5. Verbindungen nach einem der Ansprüche 1 bis 4, worin $R_4$ Wasserstoff oder Fluor und $R_5$ $C_1$-$C_4$-Alkyl oder Trifluormethyl, oder $R_4$ und $R_5$ zusammen Tri- oder Tetramethylen bedeuten.

6. Verbindungen nach Anspruch 1, ausgewählt aus

2-Cyano-5-(1,2,4,5,6,7-hexahydro-1-methyl-2,4-dioxo-3H-cyclopenta[d]pyrimidin-3-yl)-benzoesäure-isopropylester,
2-Cyano-4-fluor-5-(1,2,4,5,6,7-hexahydro-1-methyl-2,4-dioxo-3H-cyclopenta[d]-pyrimidin-3-yl)-benzoesäure-isopropylester,
2-Cyano-5-[1,4,5,6,7,8-hexahydro-1-methyl-2,4-dioxo-3(2H)-chinazolinyl]-benzoesäure-isopropylester,
2-Cyano-4-fluor-5-[1,4,5,6,7,8-hexahydro-1-methyl-2,4-dioxo-3(2H)-chinazolinyl]-benzoesäure-isopropylester,
2-Cyano-4-fluor-5-[3,6-dihydro-3,4-dimethyl-2,6-dioxo-1(2H)-pyrimidinyl]-benzoesäure-isopropylester,
2-Cyano-5-[3,6-dihydro-3-methyl-4-trifluormethyl-2,6-dioxo-1(2H)-pyrimidinyl]-benzoesäure-isopropylester und
2-Cyano-5-[3,6-dihydro-3-methyl-4-trifluormethyl-2,6-dioxo-1(2H)-pyrimidinyl]-4-fluor-benzoesäure-isopropylester.

7. Unkkrautbekämpfungsmittel, dadurch gekennzeichnet, dass es eine wirksame Menge mindestens einer Verbindung der allgemeinen Formel

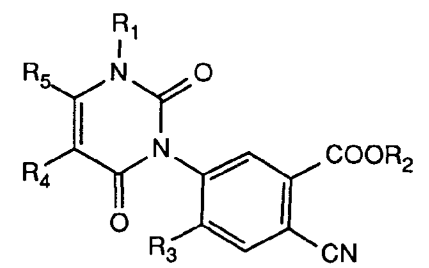

I

worin $R_1$ Wasserstoff, $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl,
$R_2$ Wasserstoff, $C_1$-$C_6$-Alkyl, $C_2$-$C_6$-Alkoxyalkyl, gegebenenfalls mit 1 bis 3 $C_1$-$C_3$-Alkylgruppen ringsubstituiertes $C_4$-$C_7$-Cycloalkenyl-$C_1$-$C_4$-alkyl, $C_3$-$C_4$-Alkenyl, gegebenenfalls mit 1 bis 3 $C_1$-$C_3$-Alkylgruppen ringsubstituiertes $C_4$-$C_7$-Cycloalkenyl-$C_3$-$C_5$-alkenyl, Aryl-$C_3$-$C_5$-alkenyl, $C_3$-$C_4$-Alkinyl, gegebenenfalls mit 1 bis 3 $C_1$-$C_3$-Alkylgruppen ringsubstituiertes $C_4$-$C_7$-Cycloalkenyl-$C_3$-$C_5$-alkinyl-alkinyl oder Aryl-$C_3$-$C_5$-alkinyl,
$R_3$ Wasserstoff oder Halogen,
$R_4$ Wasserstoff, Halogen oder $C_1$-$C_4$-Alkyl und
$R_5$ $C_1$-$C_4$-Alkyl oder $C_1$-$C_4$-Halogenalkyl, oder
$R_4$ und $R_5$ zusammen Tri- oder Tetramethylen bedeuten, mit der Massgabe, dass, falls $R_5$ für $C_1$-$C_4$-Halogenalkyl steht, $R_1$ verschieden von $C_1$-$C_4$-Halogenalkyl und $R_2$ verschieden von Wasserstoff ist,
oder eines Enoläthers einer solchen Verbindung I, in der $R_1$ von Wasserstoff oder $C_1$-$C_4$-Halogenakyl verschieden ist, oder eines Salzes einer solchen Verbindung, in der $R_1$ und/oder $R_2$ Wasserstoff bedeutet, sowie Formulierungshilfsstoffe enthält.

8. Unkrautbekämpfungsmittel nach Anspruch 7, dadurch gekennzeichnet, dass es eine wirksame Menge mindestens einer aus der Gruppe
2-Cyano-5-(1,2,4,5,6,7-hexahydro-1-methyl-2,4-dioxo-3H-cyclopenta[d]pyrimidin-3-yl)-benzoesäure-isopropylester,
2-Cyano-4-fluor-5-(1,2,4,5,6,7-hexahydro-1-methyl-2,4-dioxo-3H-cyclopenta[d]-pyrimidin-3-yl)-benzoesäure-isopropylester,
2-Cyano-5-[1,4,5,6,7,8-hexahydro-1-methyl-2,4-dioxo-3(2H)-chinazolinyl]-benzoesäure-isopropylester,
2-Cyano-4-fluor-5-[1,4,5,6,7,8-hexahydro-1-methyl-2,4-dioxo-3(2H)-chinazolinyl]-benzoesäure-isopropylester,
2-Cyano-4-fluor-5-[3,6-dihydro-3,4-dimethyl-2,6-dioxo-1(2H)-pyrimidinyl]-benzoesäure-isopropylester,
2-Cyano-5-[3,6-dihydro-3-methyl-4-trifluormethyl-2,6-dioxo-1(2H)-pyrimidinyl]-benzoesäure-isopropylester und
2-Cyano-5-[3,6-dihydro-3-methyl-4-trifluormethyl-2,6-dioxo-1(2H)-pyrimidinyl]-4-fluor-benzoesäure-isopropylester
ausgewählten Verbindung sowie Formulierungshilfsstoffe enthält.

9. Verfahren zur Herstellung von Verbindungen der allgemeinen Formel

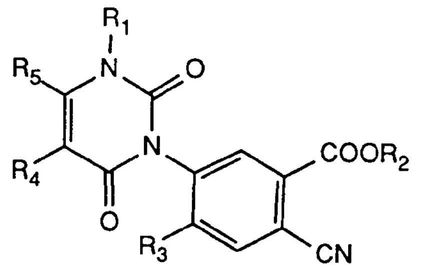

I

worin
$R_1$ Wasserstoff, $C_1$-$C_4$-Alkyl, $C_3$-$C_4$-Akenyl, $C_3$-$C_4$-Alkinyl, $C_2$-$C_6$-Alkoxyalkyl oder $C_1$-$C_4$-Halogenalkyl,
$R_2$ Wasserstoff, $C_1$-$C_6$-Alkyl, $C_2$-$C_6$-Alkoxyalkyl, gegebenenfalls mit 1 bis 3 $C_1$-$C_3$-Alkylgruppen ringsubstituiertes $C_4$-$C_7$-Cycloalkenyl-$C_1$-$C_4$-alkyl, $C_3$-$C_4$-Alkenyl, gegebenenfalls mit 1 bis 3 $C_1$-$C_3$-Alkylgruppen ringsubstituiertes $C_4$-$C_7$-Cycloalkenyl-$C_3$-$C_5$-alkenyl, Aryl-$C_3$-$C_5$-alkenyl, $C_3$-$C_4$-Alkinyl, gegebenenfalls mit 1 bis 3 $C_1$-$C_3$-Alkylgruppen ringsubstituiertes $C_4$-$C_7$-Cycloalkenyl-$C_3$-$C_5$-alkinyl oder Aryl-$C_3$-$C_5$-alkinyl,
$R_3$ Wasserstoff oder Halogen,
$R_4$ Wasserstoff, Halogen oder $C_1$-$C_4$-Alkyl und
$R_5$ $C_1$-$C_4$-Alkyl oder $C_1$-$C_4$-Halogenalkyl, oder
$R_4$ und $R_5$ zusammen Tri- oder Tetramethylen bedeuten, mit der Massgabe, dass, falls $R_5$ für $C_1$-$C_4$-Halogenalkyl steht, $R_1$ verschieden von $C_1$-$C_4$-Halogenalkyl und $R_2$ verschieden von Wasserstoff ist,
und von den Enoläthern derjenigen Verbindungen der Formel I, in denen $R_1$ von Wasserstoff oder $C_1$-$C_4$-Halogenalkyl verschieden ist, sowie von Salzen derjenigen Verbindungen der Formel I, in denen $R_1$ und-/oder $R_2$ Wasserstoff bedeutet, dadurch gekennzeichnet, dass man
a) zwecks Herstellung derjenigen Verbindungen der Formel I, in denen $R_1$ Wasserstoff bedeutet und $R_2$ verschieden von Wasserstoff und $R_4$ verschieden von Chlor, Brom oder Jod sind, sowie gewünschtenfalls von Metallsalzen dieser Verbindungen, eine Verbindung der allgemeinen Formel

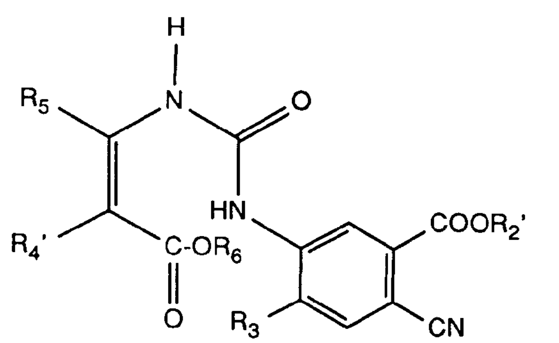

II

worin $R_3$ und $R_5$ die oben angegebenen Bedeutungen besitzen, $R_2'$ die oben angegebene Bedeutung von $R_2$ mit Ausnahme von Wasserstoff besitzt, $R_4'$ Wasserstoff, Fluor oder $C_1$-$C_4$-Akyl oder zusammen mit $R_5$ Tri- oder Tetramehtylen bedeutet und $R_6$ nieder Alkyl bedeutet,
einer Cyclisierung unter basischen Bedingungen unterwirft und gewünschtenfalls eine allfällig erhaltene Metallsalzform des Uracilderivats durch Behandlung mit einer Säure in die entsprechende saure Form ($R^1$ = Wasserstoff) überführt,
b) zwecks Herstellung derjenigen Verbindungen der Formel I, in denen $R_1$ Wasserstoff bedeutet und $R_2$ verschieden von Wasserstoff, $R_4$ verschieden von Chlor, Brom oder Jod und $R_5$ verschieden von $C_1$-$C_4$-Halogenalkyl sind, sowie gewünschtenfalls von Mettalsalzen dieser Verbindungen, eine Verbindung der allgemeinen Formel

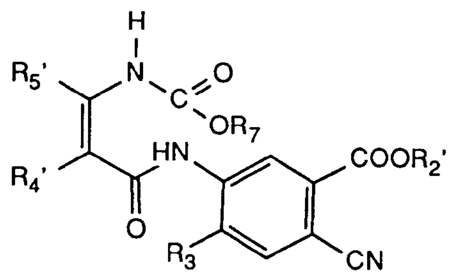

III

worin $R_2'$ und $R_3$ die oben angegebenen Bedeutungen besitzen, $R_4'$ Wasserstoff, Fluor oder $C_1$-$C_4$-Alkyl oder zusammen mit $R_5'$ Tri- oder Tetramethylen bedeutet, $R_5'$ $C_1$-$C_4$-Alkyl oder zusammen mit $R_4'$ Tri- oder Tetramehtylen bedeutet, und $R_7$ nieder Alkyl bedeutet,
einer Cyclisierung unter basischen Bedingungen unterwirft und gewünschtenfalls ein allfällig erhaltenes Metallsalz des Uracilderivats der Formel I durch Behandlung mit einer Säure in die saure Form ($R_1$ = Wasserstoff) überführt.
c) zwecks Herstellung derjenigen Verbindungen der Formel I, in denen $R_1$ $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl bedeutet, ein Uracilderivat der allgemeinen Formel

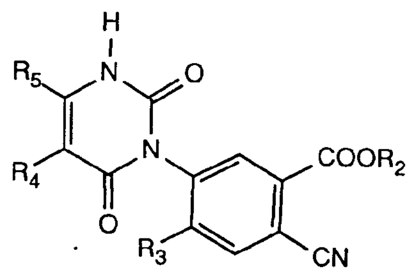

I'

worin $R_2$, $R_3$, $R_4$ und $R_5$ die oben angegebenen Bedeutungen besitzen,
einer Alkylierung mit einem entstprechenden eine $C_1$-$C_4$-Alkyl-, $C_3$-$C_4$-Alkenyl-, $C_3$-$C_4$-Alkinyl-, $C_2$-$C_6$-Alkoxyalkyl- oder $C_1$-$C_4$-Halogenalkylgruppe enthaltenden Alkylierungsmittel unterwirft,
d) zwecks Herstellung sämtlicher Verbindungen der Formel I und der Enoläther ein Uracilderivat der allgemeinen Formel

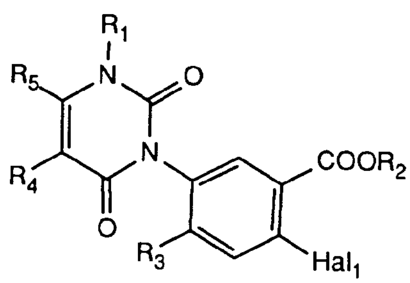

IV

worin $Hal_1$ Halogen, vorzugsweise Chlor oder Brom, bedeutet und $R_1$, $R_2$, $R_3$, $R_4$ und $R_5$ die oben angegebenen Bedeutungen besitzen,
oder den entsprechenden Enoläther mit einem Metallcyanid behandelt,
e) zwecks Herstellung derjenigen Verbindungen der Formel I, in denen $R_2$ Wasserstoff bedeutet und $R_5$ verschieden von $C_1$-$C_4$-Halogenalkyl ist, und von deren Enoläthern einen Benzoesäureester der allgemeinen Formel

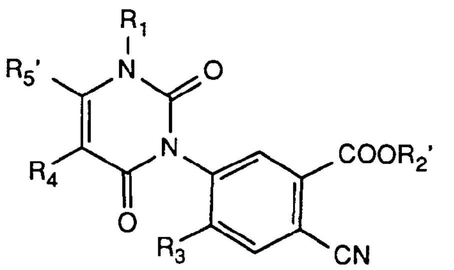

I''

worin $R_1$, $R_2'$, $R_3$, $R_4$ und $R_5'$die oben angegebenen Bedeutungen besitzen, zur entsprechenden Benzoesäure hydrolysiert,

f) zwecks Herstellung derjenigen Verbindungen der Formel I, in denen $R_1$ und $R_2$ jeweils verschieden von Wasserstoff und $R_5$ verschieden von $C_1$-$C_4$-Halogenalkyl sind, und der entsprechenden Enoläther eine Benzoesäure der allgemeinen Formel

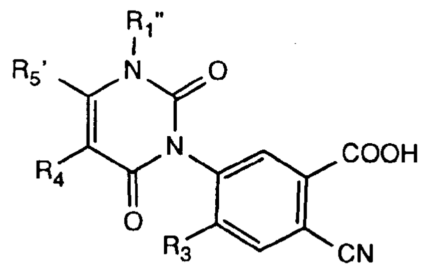

I'''

worin $R_1$ '' $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl bedeutet und $R_3$, $R_4$ und $R_5'$die oben angegebenen Bedeutungen besitzen, oder den entsprechenden Enoläther, wobei die Benzoesäure oder deren Enoläther in Form eines reaktionsfähigen Derivats vorliegen kann, mit einer Hydroxyverbindung der allgemeinen Formel

$HO$-$R_2'$ V

worin $R_2'$ die oben angegebene Bedeutung besitzt, oder mit einem reaktionsfähigen Derivat dieser Hydroxyverbindung verestert,

g) zwecks Herstellung derjenigen Verbindungen der Formel I, in denen $R_1$ und $R_2$ jeweils verschieden von Wasserstoff ist, und von deren Enoläthern einen Benzoesäureester der allgemeinen Formel

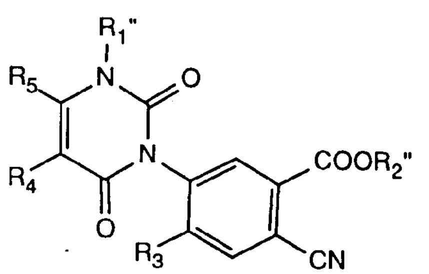

I''''

worin $R_1''$, $R_3$, $R_4$ und $R_5$ die oben angegebenen Bedeutungen besitzen und $R_2''$ $C_1$-$C_6$-Alkyl, $C_3$-$C_4$-Alkenyl, $C_3$-$C_4$-Alkinyl oder $C_2$-$C_6$-Alkoxyalkyl bedeutet, oder den entsprechenden Enoläther einer Umesterungsreaktion mit einer Hydroxyverbindung der oben angegebenen Formel V unterwirft, wobei das Reagens V höher siedend ist als das Alkanol, Akenol bzw. Alkinol $R_2''$ OH,

h) zwecks Herstellung derjenigen Verbindungen der Formel I, in denen $R_4$ Chlor, Brom oder Jod bedeutet, und der entsprechenden Enoläther ein Uracilderivat der allgemeinen Formel

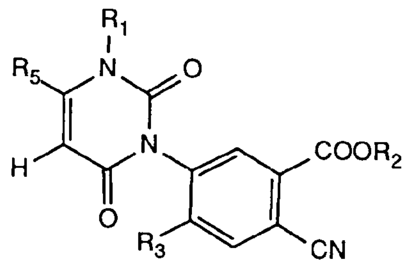

I'''''

worin $R_1$, $R_2$, $R_3$ und $R_5$ die oben angegebenen Bedeutungen besitzen, oder den entsprechenden Enoläther chloriert, bromiert bzw. jodiert, i) zwecks Herstellung der Enoläther der Verbindungen der Formel I, ein Uracilderivat der allgemeinen Formel

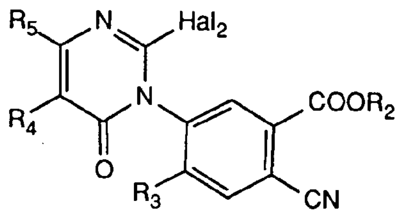

VI

worin $R_2$, $R_3$, $R_4$ und $R_5$ die oben angegebenen Bedeutungen besitzen und $Hal_2$ Chlor oder Brom bedeutet, mit einem Alkanol, Alkenol oder Alkinol $R_1'OH$ in Gegenwart einer organischen Base oder mit dem entsprechenden Alkoholat, Alkenolat bzw. Alkinolat der allgemeinen Formel

$$R_1'O^{\ominus}M^{\oplus} \qquad VII$$

worin $R_1'$die oben angegebene Bedeutung besitzt und
$M^{\oplus}$ ein Aequivalent eines Metallions bedeutet,
behandelt, und gewünschtenfalls eine so erhaltene Verbindung der Formel I, in der $R_1$ und/oder $R_2$ Wasserstoff bedeutet, in ein Salz überführt.

**10.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1 bis 6 bzw. eines Mittels gemäss Anspruch 7 oder 8 behandelt.

**11.** Verwendung einer Verbindung gemäss einem der Ansprüche 1 bis 6 bzw. eines Mittels gemäss Anspruch 7 oder 8 zur Bekämpfung von Unkräutern.

**Patentansprüche für folgenden Vertragsstaat : ES**

**1.** Verfahren zur Herstellung von Verbindungen der allgemeinen Formel

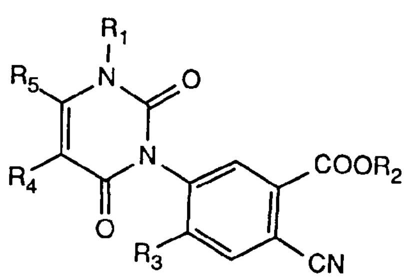

I

worin
$R_1$ Wasserstoff, $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl,
$R_2$ Wasserstoff, $C_1$-$C_6$-Alkyl, $C_2$-$C_6$-Alkoxyalkyl, gegebenenfalls mit 1 bis 3 $C_1$-$C_3$-Alkylgruppen ringsubstituiertes $C_4$-$C_7$-Cycloalkenyl-$C_1$-$C_4$-alkyl, $C_3$-$C_4$-Alkenyl, gegebenenfalls mit 1 bis 3 $C_1$-$C_3$-Alkylgruppen

ringsubstituiertes $C_4$-$C_7$-Cycloalkenyl-$C_3$-$C_5$-alkenyl, Aryl-$C_3$-$C_5$-alkenyl, $C_3$-$C_4$-Alkinyl, gegebenenfalls mit 1 bis 3 $C_1$-$C_3$-Alkylgruppen ringsubstituiertes $C_4$-$C_7$-Cycloalkenyl-$C_3$-$C_5$-alkinyl oder Aryl-$C_3$-$C_5$-alkinyl,
$R_3$ Wasserstoff oder Halogen,
$R_4$ Wasserstoff, Halogen oder $C_1$-$C_4$-Alkyl und
$R_5$ $C_1$-$C_4$-Alkyl oder $C_1$-$C_4$-Halogenalkyl, oder
$R_4$ und $R_5$ zusammen Tri- oder Tetramethylen bedeuten, mit der Massgabe, dass, falls $R_5$ für $C_1$-$C_4$-Halogenalkyl steht, $R_1$ verschieden von $C_1$-$C_4$-Halogenalkyl und $R_2$ verschieden von Wasserstoff ist,
und von den entsprechenden Enoläther derjenigen Verbindungen der Formel I, in denen $R_1$ von Wasserstoff oder $C_1$-$C_4$-Halogenalkyl verschieden ist, sowie Salze derjenigen Verbindungen der Formel I, in denen $R_1$ und/oder $R_2$ Wasserstoff bedeutet, dadurch gekennzeichnet, dass man
a) zwecks Herstellung derjenigen Verbindungen der Formel I, in denen $R_1$ Wasserstoff bedeutet und $R_2$ verschieden von Wasserstoff und $R_4$ verschieden von Chlor, Brom oder Jod sind, sowie gewünschtenfalls von Metallsalzen dieser Verbindungen, eine Verbindung der allgemeinen Formel

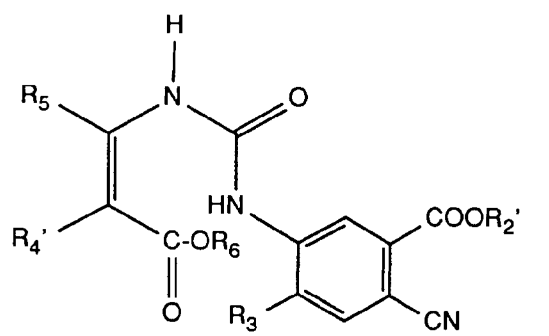

II

worin $R_3$ und $R_5$ die unter Formel I angegebenen Bedeutungen besitzen, $R_2$'die unter Formel I angegebene Bedeutung von $R_2$ mit Ausnahme von Wasserstoff besitzt, $R_4'$ Wasserstoff, Fluor oder $C_1$-$C_4$-Alkyl oder zusammen mit $R_5$ Tri- oder Tetramethylen bedeutet und $R_5$ nieder Alkyl bedeutet,
einer Cyclisierung unter basischen Bedingungen unterwirft und gewünschtenfalls eine allfällig erhaltene Metallsalzform des Uracilderivats durch Behandlung mit einer Säure in die entsprechende saure Form ($R^1$ = Wasserstoff) überführt,
b) zwecks Herstellung derjenigen Verbindungen der Formel I, in denen $R_1$ Wasserstoff bedeutet und $R_2$ verschieden von Wasserstoff, $R_4$ verschieden von Chlor, Brom oder Jod und $R_5$ verschieden von $C_1$-$C_4$-Halogenalkyl sind, sowie gewünschtenfalls von Metallsalzen dieser Verbindungen, eine Verbindung der allgemeinen Formel

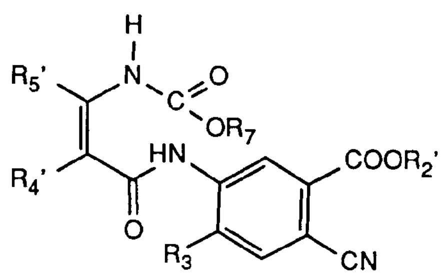

III

worin $R_2$'und $R_3$ die unter Formel I angegebenen Bedeutungen besitzen, $R_4'$ Wasserstoff, Fluor oder $C_1$-$C_4$-Alkyl oder zusammen mit $R_5'$ Tri- oder Tetramethylen bedeutet, $R_5'$ $C_1$-$C_4$-Alkyl oder zusammen mit $R_4'$ Tri- oder Tetramethylen bedeutet, und $R_7$ nieder Alkyl bedeutet,
einer Cyclisierung unter basischen Bedingungen unterwirft und gewünschtenfallsein allfällig erhaltenes Metallsalz des Uracilderivats der Formel I durch Behandlung mit einer Säure in die saure Form ($R_1$ = Wasserstoff) überführt,
c) zwecks Herstellung derjenigen Verbindungen der Formel I, in denen $R_1$ $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl bedeutet, ein Uracilderivat der allgemeinen Formel

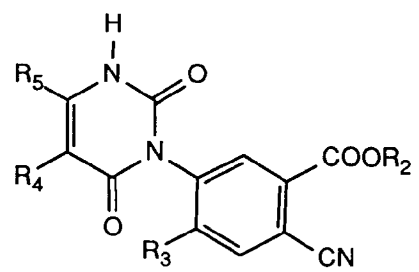

I'

worin $R_2$, $R_3$, $R_4$ und $R_5$ die unter Formel I angegebenen Bedeutungen besitzen, einer Alkylierung mit einem entstprechenden eine $C_1$-$C_4$-Alkyl-, $C_3$-$C_4$-Alkenyl-, $C_3$-$C_4$-Alkinyl-, $C_2$-$C_6$-Alkoxyalkyl- oder $C_1$-$C_4$-Halogenalkylgruppe enthaltenden Alkylierungsmittel unterwirft,

d) zwecks Herstellung sämtlicher Verbindungen der Formel I und der Enoläther ein Uracilderivat der allgemeinen Formel

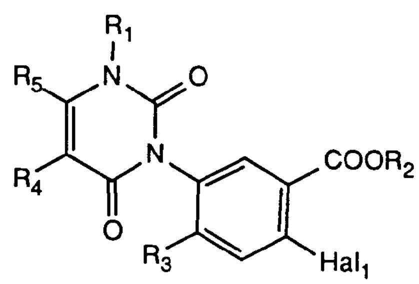

IV

worin $Hal_1$ Halogen, vorzugsweise Chlor oder Brom, bedeutet und $R_1$, $R_2$, $R_3$, $R_4$ und $R_5$ die unter Formel I angegebenen Bedeutungen besitzen, oder den entsprechenden Enoläther mit einem Metallcyanid behandelt,

e) zwecks Herstellung derjenigen Verbindungen der Formel I, in denen $R_2$ Wasserstoff bedeutet und $R_5$ verschieden von $C_1$-$C_4$-Halogenalkyl ist, und von deren Enoläthern einen Benzoesäureester der allgemeinen Formel

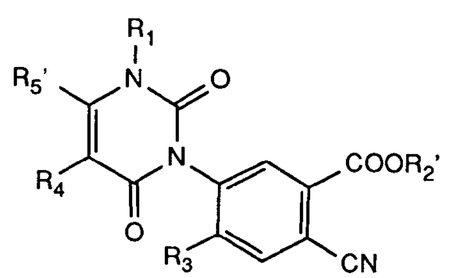

I"

worin $R_1$, $R_2'$, $R_3$, $R_4$ und $R_5'$ die unter Formel I angegebenen Bedeutungen besitzen, zur entsprechenden Benzoesäure hydrolysiert,

f) zwecks Herstellung derjenigen Verbindungen der Formel I, in denen $R_1$ und $R_2$ jeweils verschieden von Wasserstoff und $R_5$ verschieden von $C_1$-$C_4$-Halogenalkyl sind, und der entsprechenden Enoläther eine Benzoesäure der allgemeinen Formel

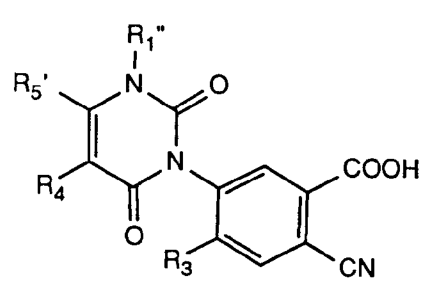

I'''

worin $R_1''$ $C_1$-$C_4$-Alkyl, $C_3$-$C_4$-Alkenyl, $C_3$-$C_4$-Alkinyl, $C_2$-$C_6$-Alkoxyalkyl oder $C_1$-$C_4$-Halogenalkyl bedeutet und $R_3$, $R_4$ und $R_5'$ die unter Formel I angegebenen Bedeutungen besitzen,
oder den entsprechenden Enoläther, wobei die Benzoesäure oder deren Enoläther in Form eines reaktionsfähigen Derivats vorliegen kann, mit einer Hydroxyverbindung der allgemeinen Formel

$$HO\text{-}R_2' \qquad V$$

worin $R_2'$ die unter Formel I angegebene Bedeutung besitzt,
oder mit einem reaktionsfähigen Derivat dieser Hydroxyverbindung verestert,
g) zwecks Herstellung derjenigen Verbindungen der Formel I, in denen $R_1$ und $R_2$ jeweils verschieden von Wasserstoff ist, und von deren Enoläthern einen Benzoesäureester der allgemeinen Formel

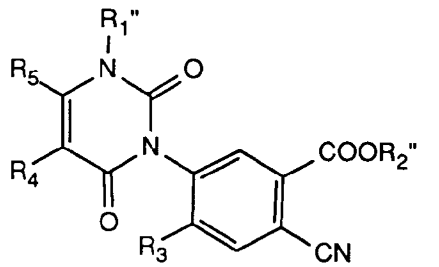

I''''

worin $R_1''$, $R_3$, $R_4$ und $R_5$ die unter Formel I angegebenen Bedeutungen besitzen und $R_2''$ $C_1$-$C_6$-Alkyl, $C_3$-$C_4$-Akenyl, $C_3$-$C_4$-Alkinyl oder $C_2$-$C_6$-Alkoxyalkyl bedeutet,
oder den entsprechenden Enoläther einer Umesterungsreaktion mit einer Hydroxyverbindung der unter Formel I angegebenen Formel V unterwirft, wobei das Reagens V höher siedend ist als das Alkanol, Alkenol bzw. Alkinol $R_2''$ OH,
h) zwecks Herstellung derjenigen Verbindungen der Formel I, in denen $R_4$ Chlor, Brom oder Jod bedeutet, und der entsprechenden Enoläther ein Uracilderivat der allgemeinen Formel

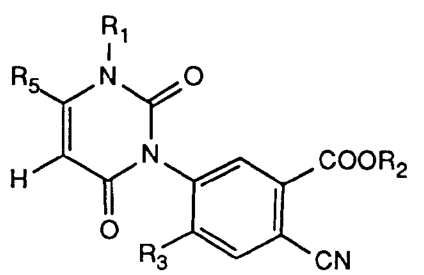

I'''''

worin $R_1$, $R_2$, $R_3$ und $R_5$ die unter Formel I angegebenen Bedeutungen besitzen,
oder den entsprechenden Enoläther chloriert, bromiert bzw. jodiert,
i) zwecks Herstellung der Enoläther der Verbindungen der Formel I, ein Uracilderivat der allgemeinen Formel

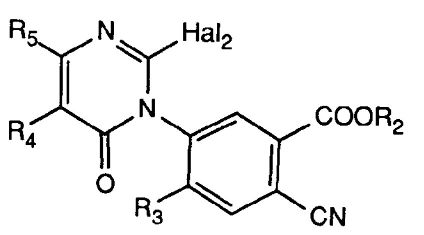

VI

worin $R_2$, $R_3$, $R_4$ und $R_5$ die unter Formel I angegebenen Bedeutungen besitzen und $Hal_2$ Chlor oder Brom bedeutet,
mit einem Alkanol, Alkenol oder Alkinol $R_1'OH$ in Gegenwart einer organischen Base oder mit dem entsprechenden Alkoholat, Alkenolat bzw. Alkinolat der allgemeinen Formel

$$R_1'O^{\ominus}M^{\oplus} \qquad VII$$

worin $R_1'$ die unter Formel I angegebene Bedeutung besitzt und

$M^{\oplus}$ ein Aequivalent eines Metallions bedeutet,
behandelt, und gewünschtenfalls eine so erhaltene Verbindung der Formel I, in der $R_1$ und/oder $R_2$ Wasserstoff bedeutet, in ein Salz überführt..

2. Verfahren nach Anspruch 1 zur Herstellung von Verbindungen der Formel I, worin $R_1$ $C_1$-$C_4$-Alkyl bedeutet.

3. Verfahren nach Anspruch 1 zur Herstellung von Verbindungen der Formel I, worin $R_2$ $C_1$-$C_6$-Alkyl, $C_2$-$C_6$-Alkoxyalkyl, $C_3$-$C_4$-Alkenyl oder $C_3$-$C_4$-Alkinyl bedeutet.

4. Verfahren nach Anspruch 1 zur Herstellung von Verbindungen der Formel I worin $R_3$ Wasserstoff oder Fluor bedeutet.

5. Verfahren nach Anspruch 1 zur Herstellung von Verbindungen der Formel I, worin $R_4$ Wasserstoff oder Fluor und $R_5$ $C_1$-$C_4$-Alkyl oder Trifluormethyl, oder $R_4$ und $R_5$ zusammen Tri- oder Tetramethylen bedeuten.

6. Verfahren nach Anspruch 1 zur Herstellung von
2-Cyano-5-(1,2,4,5,6,7-hexahydro-1-methyl-2,4-dioxo-3H-cyclopenta[d]pyrimidin-3-yl)-benzoesäure-isopropylester,
2-Cyano-4-fluor-5-(1,2,4,5,6,7-hexahydro- 1-methyl-2,4-dioxo-3H-cyclopenta[d]-pyrimidin-3-yl)-benzoesäure-isopropylester,
2-Cyano-5-[1,4,5,6,7,8-hexahydro-1-methyl-2,4-dioxo-3(2H)-chinazolinyl]-benzoesäure-isopropylester,
2-Cyano-4-fluor-5-[1,4,5,6,7,8-hexahydro-1-methyl-2,4-dioxo-3(2H)-chinazolinyl]-benzoesäure-isopropylester,
2-Cyano-4-fluor-5-[3,6-dihydro-3,4-dimethyl-2,6-dioxo-1(2H)-pyrimidinyl]-benzoesäure-isopropylester,
2-Cyano-5-[3,6-dihydro-3-methyl-4-trifluormethyl-2,6-dioxo-1(2H)-pyrimidinyl]-benzoesäure-isopropylester und
2-Cyano-5-[3,6-dihydro-3-methyl-4-trifluormethyl-2,6-dioxo-1-(2H)-pymidinyl]-4-fluor-benzoesäure-isopropylester.

7. Verwendung einer Verbindung der Formel I gemäss Anspruch 1 zur Bekämpfung von Unkräutern.

Europäisches Patentamt

# EUROPÄISCHER RECHERCHENBERICHT

Nummer der Anmeldung

EP 91 81 0671

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| --- | --- | --- | --- |
| A | WO-A-88010254 (F.HOFFMANN-LA ROCHE & CO AG)<br>* Ansprüche 1, 14, 15, 19-21, 23, 24 *<br>--- | 1, 7, 9-11 | C07D239/54<br>C07D239/52<br>C07D239/96<br>C07D239/70<br>A01N43/54 |
| A | WO-A-89003825 (F. HOFFMANN-LA ROCHE & CO AG)<br>* Ansprüche 1, 8, 11, 13, 14 *<br>--- | 1, 7, 9-11 | |
| A,D | EP-A-195346 (F. HOFFMANN-LA ROCHE & CO AG)<br>* Ansprüche 1, 2, 18, 21, 23, 25 *<br>--- | 1, 7, 9-11 | |
| A | EP-A-255047 (F. HOFFMANN-LA ROCHE & CO AG)<br>* Ansprüche 1, 24, 29, 30, 32 *<br>--- | 1, 7, 9-11 | |
| A,D | EP-A-260621 (F. HOFFMANN-LA ROCHE & CO AG)<br>* Ansprüche 1, 15, 20, 22, 24 *<br>----- | 1, 7, 9-11 | |
| | | | RECHERCHIERTE SACHGEBIETE (Int. Cl.5 ) |
| | | | C07D239/00<br>A01N43/00 |

Der vorliegende Recherchenbericht wurde für alle Patentansprüche erstellt

| Recherchenort | Abschlußdatum der Recherche | Prüfer |
| --- | --- | --- |
| BERLIN | 21 NOVEMBER 1991 | VAN AMSTERDAM L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)